# EUROPEAN PATENT SPECIFICATION

(11) **EP 0 351 029 B1**
(45) Date of publication and mention of the grant of the patent: **06.03.2002**
(21) Application number: 89201892.0
(22) Date of filing: 17.07.1989
(51) Int. Cl.: C12N 9/92, C12N 15/61, C12N 11/00

(54) **Novel glucose isomerase enzymes and their use**
Glucose-Isomerase-Enzyme und deren Verwendung
Nouvelles glucose isomérases et leur utilisation

(30) Priority: 15.07.1988 EP 88201539; 04.11.1988 EP 88402789
(43) Date of publication of application: 17.01.1990
(73) Proprietor: GENENCOR INTERNATIONAL, INC., Palo Alto, California 94304 (US); PLANT GENETIC SYSTEMS, N.V., 1040 Brussel (BE)
(72) Inventor: Luiten, Rudolf Gijsbertus Marie, NL-2317 KR Leiden (NL); Quax, Wilhelmus Johannes, NL-2253 VB Voorschoten (NL); Schuurhuizen, Paul William, NL-2623 NT Delft (NL); Mrabet, Nadir, B-1080 Koekelberg (BE)
(74) Representative: Brandes, Jürgen, Dr.

(56) References cited:
- EP-A- 0 028 942
- WO-A-89/01520
- US-A- 4 410 627
- PROTEIN ENGINEERING, vol. 1, no. 3, 5th-8th April 1987, page 264, abstract no. 154, Oxford, GB; P.-C. SHAW et al.: "Protein engineering of Arthrobacter glucose isomerase"
- BIOLOGICAL ABSTRACTS, vol. 87, no. 6, 1989, abstract no. 63577, Biological Abstracts, Inc., Philadelphia, PA, US; F. REY et al.: "Structural analysis of the 2.8A model of xylose isomerase from Actinoplanes missouriensis", & PROTEINS STRUCT. FUNCT. GENET 4(3): 165-172,1988

## Description

### TECHNICAL FIELD

The present invention relates to novel glucose isomerases, which are suitable for application in industrial processes, especially the conversion of glucose into fructose. The invention relates also to the use of these novel enzymes in the production of fructose syrups, in particular high fructose corn syrups.

### BACKGROUND OF THE INVENTION

Glucose isomerase catalyzes the reversible isomerization of glucose to fructose. Fructose is nowadays commonly applied as sugar substitute due to its higher sweetness compared to e.g. sucrose and glucose. Many microorganisms are known to produce glucose isomerase, see for example the review articles by Wen-Pin Chen in Process Biochemistry, 15 June/July (1980) 30-41 and August/September (1980) 36-41, in which a large number of microorganisms, capable of producing glucose isomerase, are listed.

Several microorganisms have been applied industrially. The Wen-Pin Chen reference describes culture conditions of the microorganisms and recovery and purification methods of the produced glucose isomerase.

The production of glucose isomerase, which is an intracellular enzyme, is relatively expensive. Special formulations have been developed to enable repeated and continuous use of the enzyme. By immobilizing the enzyme, usually in water-insoluble form, it can be used both in batch and continuous processes (e.g. packed-bed reactors). One of the major drawbacks of immobilization of glucose isomerase is the substantial decrease of specific activity, due to the the presence of inert material. The situation becomes even worse during application, since glucose isomerase is inactivated at elevated temperatures. An irreversible loss of activity will be the result of the heat-induced deterioration.

Despite efforts to retain enzyme stability substantial activity loss is still encountered under normal application conditions. There is, therefore, a continuous need for new enzymes such as glucose isomerase with improved properties. Improved thermostability of glucose isomerase, for example, will allow to take advantage of the fact that the equilibrium of the isomerisation is shifted towards fructose at higher temperatures. Most glucose isomerases are applied at pH 7.5. However, fructose is not stable at this pH. Therefore, there is also a need for glucose isomerases which can be applied below pH 7.5.

Enzymes with improved properties can be developed or found in several ways, for example by classical screening methods, by chemical modification of existing proteins, or by using modern genetic and protein engineering techniques.

Screening for organisms or microorganisms that display the desired enzymatic activity, can be performed for example by isolating and purifying the enzyme from a microorganism or from a culture supernatant of such microorganisms, determining its biochemical properties and checking whether these biochemical properties meet the demands for application.

If the identified enzyme cannot be obtained from its natural producing organism, recombinant-DNA techniques may be used to isolate the gene encoding the enzyme, express the gene in another organism, isolate and purify the expressed enzyme and test whether it is suitable for the intended application.

Modification of existing enzymes can be achieved inter alia by chemical modification methods. See, for example, I. Svendsen, Carlsberg Res. Commun. 44 (1976), 237-291. In general, these methods are too unspecific in that they modify all accessible residues with common side chains, or they are dependent on the presence of suitable amino acids to be modified, and often they are unable to modify amino acids difficult to reach, unless the enzyme molecule is unfolded.

Enzyme modification through mutagenesis of the encoding gene does not suffer from the aspecificities mentioned above, and therefore is thought to be superior. Mutagenesis can be achieved either by random mutagenesis or by site-directed mutagenesis.

Random mutagenesis, by treating whole microorganisms with chemical mutagens or with mutagenizing radiation, may of course result in modified enzymes, but then strong selection protocols are necessary to search for mutants having the desired properties. Higher probability of isolating desired mutant enzymes by random mutagenesis can be achieved by cloning the encoding gene, mutagenizing it in vitro or in vivo and expressing the encoded enzyme by recloning of the mutated gene in a suitable host cell. Also in this case suitable biological selection protocols must be available in order to select the desired mutant enzymes. These biological selection protocols do not specifically select enzymes suited for application in the fructose production.

Site-directed mutagenesis (SDM) is the most specific way of obtaining modified enzymes, enabling specific substitution of one or more amino acids by any other desired amino acid.

### SUMMARY OF THE INVENTION

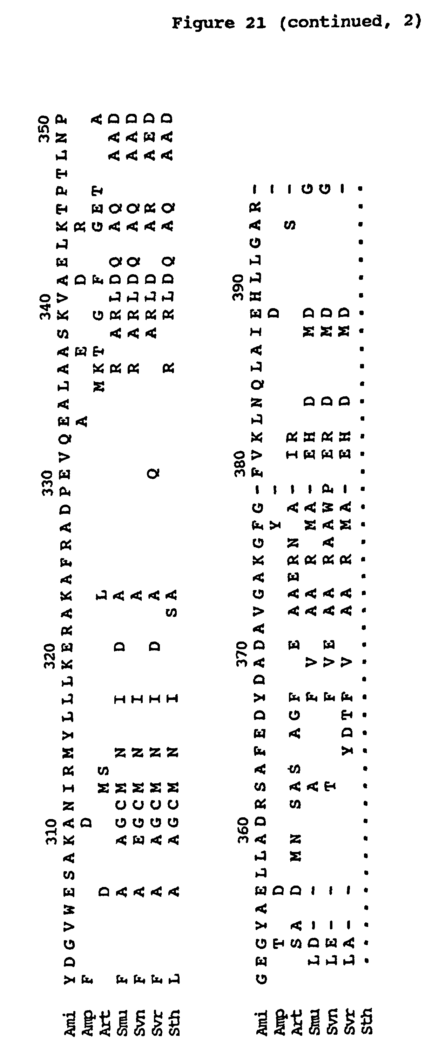

In one aspect of the present invention, a modified glucose isomerase enzyme is provided, in which the interaction between glucose isomerase subunits is enhanced, wherein said modified glucose isomerase comprises an amino acid sequence derived from a glucose isomerase wild-type sequence in which at least one of the following amino acid substitution(s) has been performed: Lys253Arg and Gly70Ser;Ala73Ser;Gly74Thr, the amino acid positions corresponding to the numbering adopted for the amino acid sequence alignment of glucose isomerase enzymes given in Figure 21.

In another aspect of the invention, an immobilized glucose isomerase comprising a modified glucose isomerase enzyme is provided.

Another aspect of the invention relates to the use of a modified glucose isomerase enzyme in the production of fructose syrups.

### BRIEF DESCRIPTION OF THE DRAWINGS

Figure 1. Heat inactivation kinetics of metal-free glucose isomerase of EcoAmi(DSM) GI in 50 mM MOPS, pH 7.2 at 25°C. No metal was added.
Figure 2. Same as Figure 1. 10 mM Mg²⁺ was added.
Figure 3. Same as Figure 1. 10 mM Co²⁺ was added.
Figure 4. Arrhenius plot of the temperature dependence for heat inactivation of EcoAmi(DSM) GI in 50 mM MOPS, pH 7.2 at 25°C.
Figure 5. pH dependence of heat inactivation of metal-free EcoAmi(DSM) GI at 72°C in the absence of added metal. CHES = 2-(cyclohexylamino) ethane sulfonic acid.
Figure 6. Ionic strength effect on the kinetics of heat inactivation of metal-free EcoAmi(DSM) GI in 50 mM MOPS, pH 6.7, 72°C. No metal added.
Figure 7. Ionic strength effect on the heat inactivation kinetics of metal-free EcoAmi(DSM) GI in 50 mM MOPS, pH 7.6, 72°C. No metal added.
Figure 8. SEC-HPLC of EcoAmi(DSM) GI after prolonged incubation of EcoAmi(DSM) GI in 7M urea at 25°C.
Figure 9A. SEC-HPLC of EcoAmi(DSM) GI pretreated with cyanate at 25°C in the absence of urea. Elution buffer was 50 mM Tris/HCl, pH 8.0, 150 mM NaCl, 0.02% NaN₃.
Figure 9B. Native PAGE of EcoAmi(DSM) GI treated with cyanate at 25°C in the absence of urea.
Figure 10A. SEC-HPLC of EcoAmi(DSM) GI pretreated with cyanate in the presence of 5M urea at 25°C.
Figure 10B. Native PAGE of EcoAmi(DSM) GI treated with cyanate in the presence of 5M urea at 25°C.
Figure 11. Glycation of EcoAmi(DSM) GI in 50 mM MOPS, pH 7.7, 60°C.
   Open circles: no glucose added; Closed circles: +250 mM glucose; Triangles: incubation with glucose (250 mM) was followed by extensive dialysis to test for reversibility.
Figure 12. Heat inactivation kinetics of EcoAmi(DSM) GI-mutant K253Q.
Figure 13. Kinetics of tetramer-dimer dissociation at 25°C in 5M urea of EcoAmi(DSM) GI mutant K253R.
Figure 14. Kinetics of the glycation-induced inactivation of EcoAmi(DSM) GI mutant K253R at 60°C in 12.5 mM Potassium phosphate, pH 7.7.
Figure 15A. Structure of pMa/c5-8.
   In the pMa type vector nucleotide 3409 is changed from A to G, while in the pMc type vector nucleotide 2238 is changed from G to C, creating amber stopcodons in the chloramphenicol acetyl transferase gene and the β-lactamase gene, respectively, rendering said genes inactive (P. Stanssens et al. (1987) in "Oligonucleotide-directed construction of mutations by the gapped duplex DNA method using the pMa/c phasmid vectors", Manual used at the EMBO laboratory course, "Directed Mutagenesis and Protein Engineering" held at the Max Planck Institut für Biochemie, Martinsried, July 4-18, 1987).
   The sequence displayed in the Figure is that of pMa5-8.

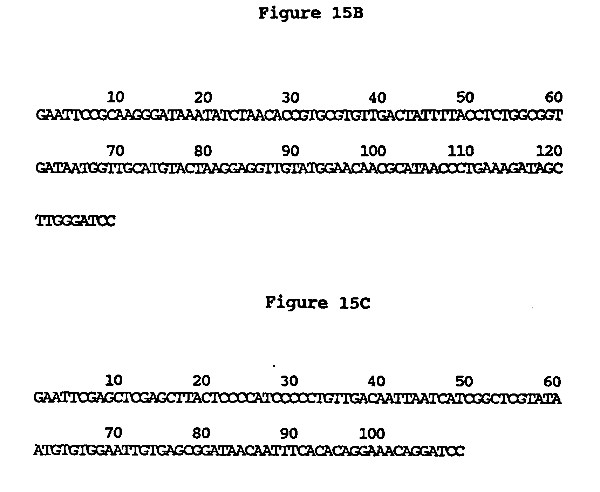

Figure 15B. Lambda P_{R} promoter sequence as present in the expression vector pMa/c5P_{R} (replacing nucleotides 3754 to 3769 of pMa/c5-8).
Figure 15C. Tac promoter sequence as present on the expression vector pMa/c5T (replacing nucleotides 3754 to 3769 of pMa/c5-8).

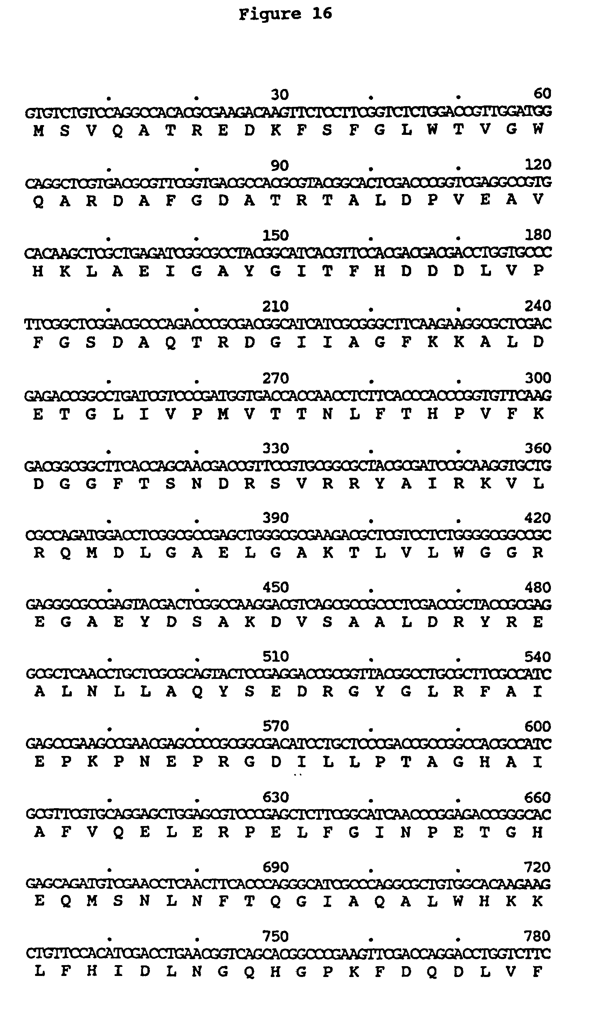

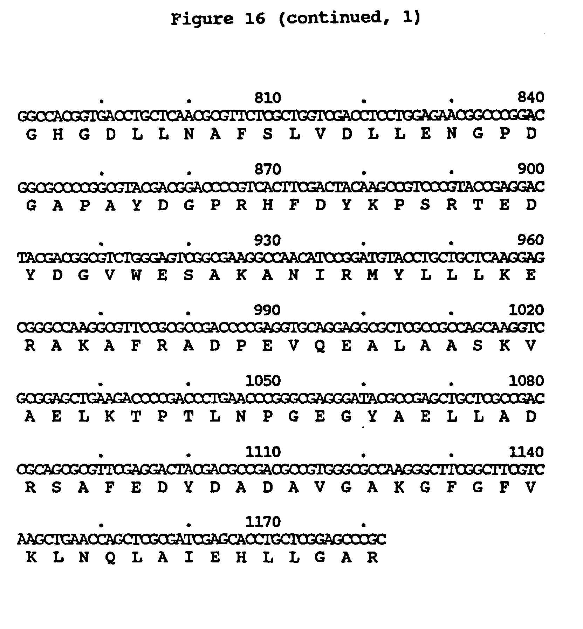

Figure 16. The complete gene sequence and derived amino acid sequence of wildtype Actinoplanes missouriensis glucose isomerase.

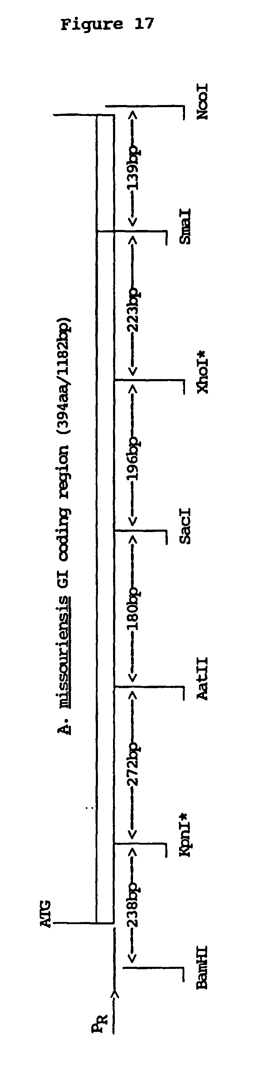

Figure 17. Physical map of the glucose isomerase (GI) expression unit on pMa/c-I. The positions of relevant restriction sites, the P_{R} promoter and the protein coding region are indicated. Asterisks indicate restriction sites introduced by site-directed mutagenesis.

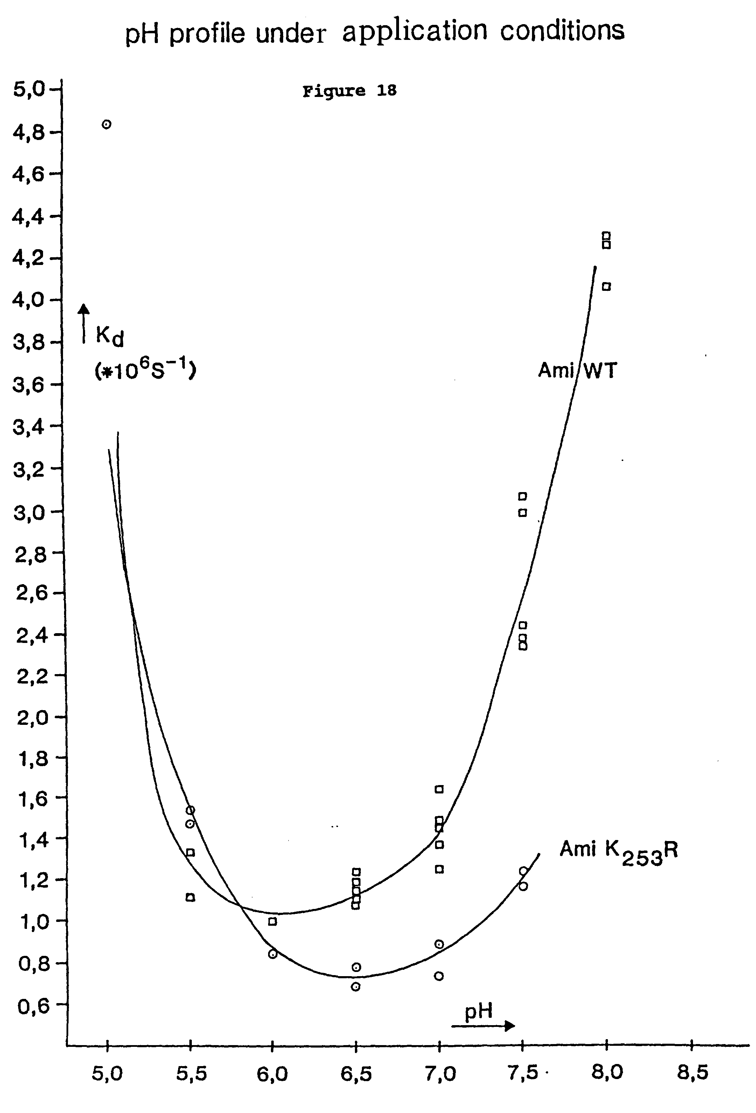

Figure 18. Properties of glucose isomerase mutant at various pH.
   - AmiWT :: wildtype A. missouriensis glucose isomerase produced from plasmid pMa-I.
   - AmiK253R :: mutant K253R glucose isomerase produced from mutated plasmid pMa-I.K253R.
   Separate k_{d} measurements for AmiWT ([]) and AmiK253R (o) are plotted. It can be seen that AmiK253R has an improved k_{d} value over a wide pH range.

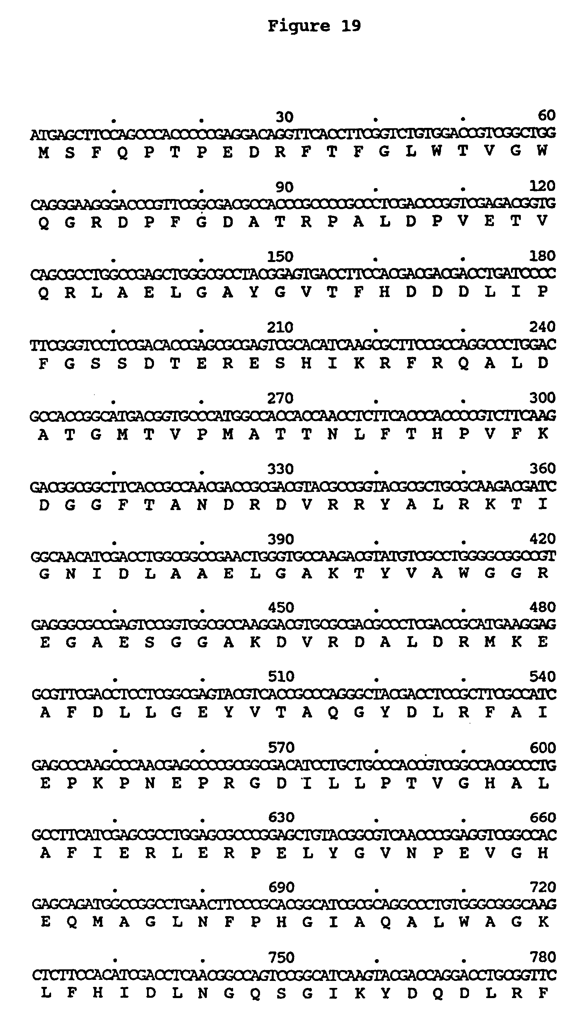

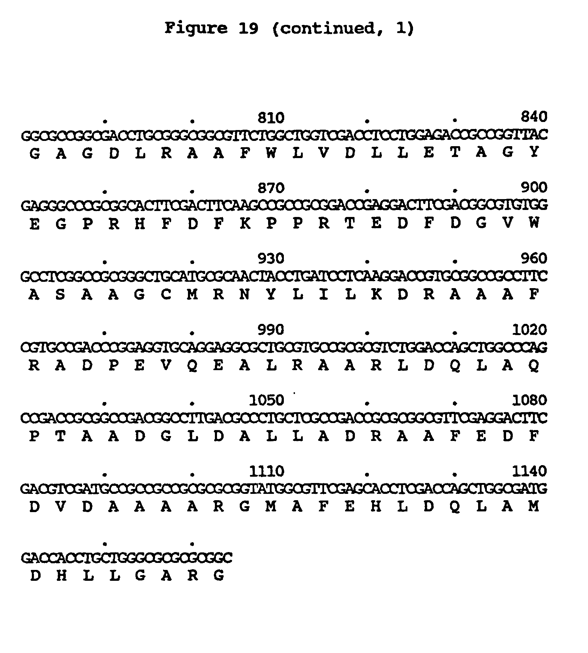

Figure 19. The complete gene sequence and derived amino acid sequence of wildtype Streptomyces murinus glucose isomerase.

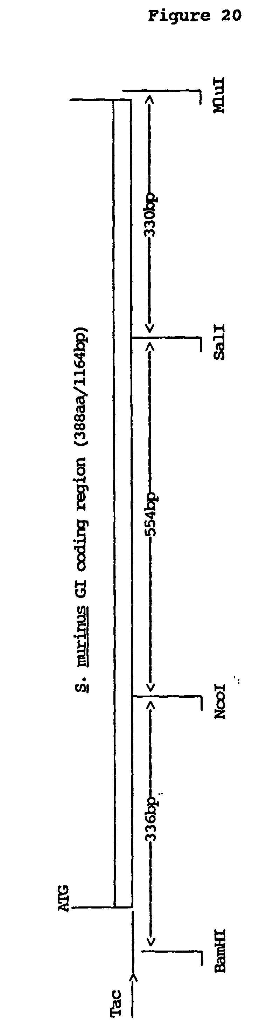

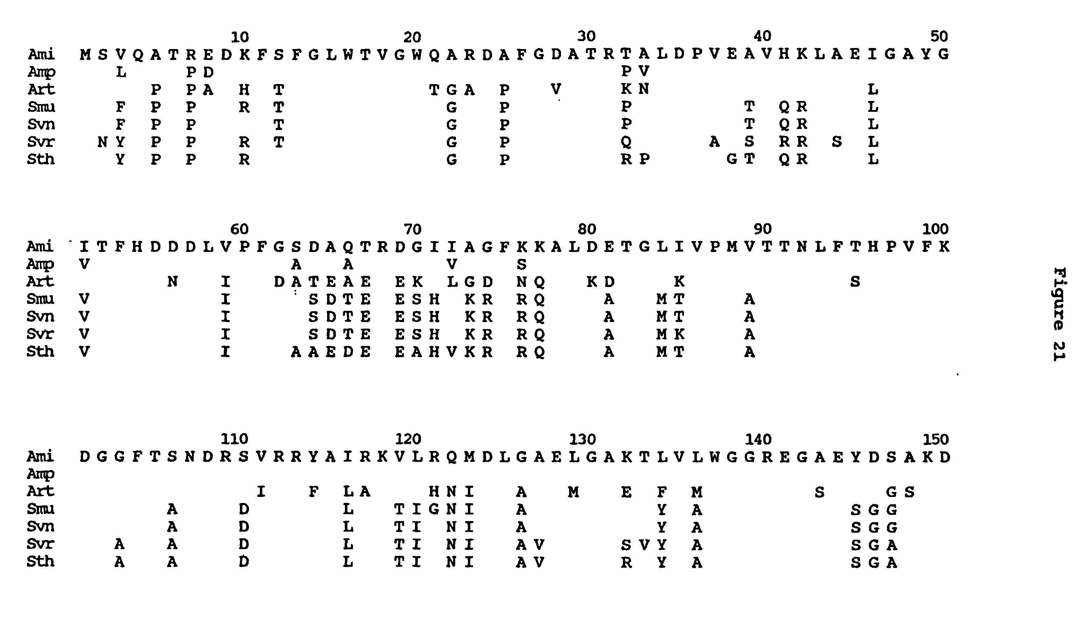

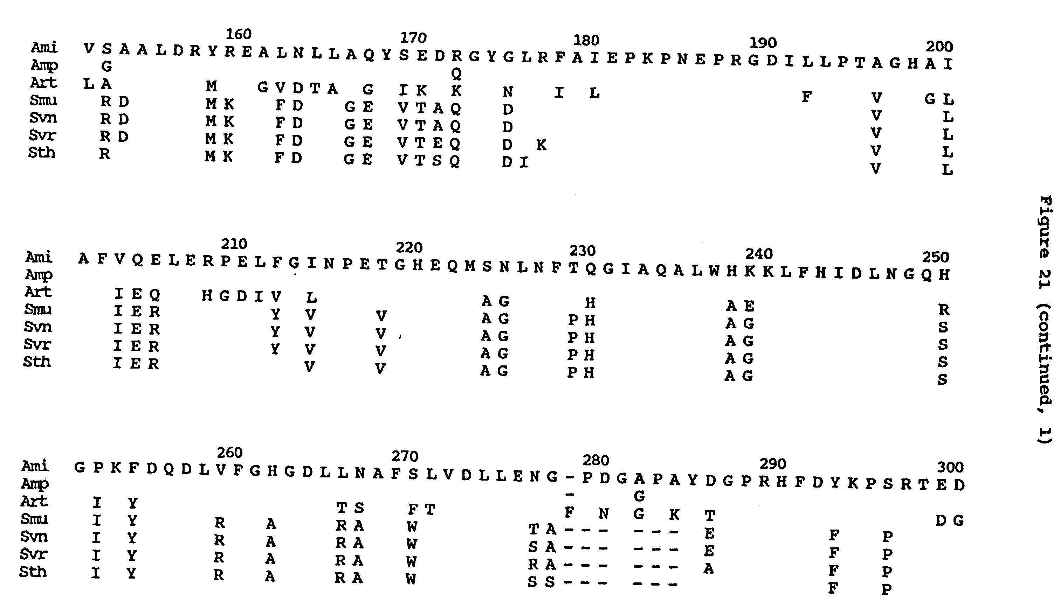

Figure 20. Physical map of the glucose isomerase (GI) expression unit on pMa/c-GIsmu1. The positions of relevant restriction sites, the Tac promoter and the protein coding region are indicated.
Figure 21. Alignment of amino acid sequences of glucose isomerases from different sources. The complete sequence of the Actinoplanes missouriensis glucose isomerase is given whereas for the glucose isomerases from other sources only the amino acid residues differing from those of Actinoplanes missouriensis glucose isomerase are shown. The amino acid sequence of the Ampullariella glucose isomerase differs from that of the published sequence (Saari, J. Bacteriol., 169 (1987) 612) by one residue: Proline 177 in the published sequence was found to be an Arginine.
   The Streptomyces thermovulgaris sequence has only been established upto amino acid 346; undetermined residues are indicated by a block (■).
   A dash (-) indicates the absence of an amino acid residue at this position as compared to any of the other sequences. The different species are indicated by the following symbols:
   - Ami :: Actinoplanes missouriensis DSM 4643
   - Amp :: Ampullariella species ATCC 31351
   - Art :: Arthrobacter species
   - Smu :: Streptomyces murinus DSM 40091
   - Svn :: Streptomyces violaceoniger CBS 409.73
   - Svr :: Streptomyces violaceoruber LMG 7183
   - Sth :: Streptomyces thermovulgaris DSM 40444

### DETAILED DESCRIPTION OF THE INVENTION

According to this invention mutant glucose isomerase enzymes can be designed based on a careful examination of the structure of wildtype glucose isomerases, combined with careful biochemical investigation of the process leading to inactivation of the original glucose isomerases, under application conditions, followed by a rational modification of the wildtype gene sequence. Extensive investigation of designed mutants under industrial application conditions has resulted in the identification of mutants with improved properties.

By "improved properties" as used herein in connection with the present glucose isomerase enzymes, we mean higher conversion performance and/or improved stability, especially heat stability, relative to the corresponding wildtype enzymes. In addition, increased stability at different pH as such or in combination with enhanced thermostability is considered within the term "improved properties".

The present invention is based on the discovery that the activity of certain enzymes is optimal when the enzyme is in multimeric (dimeric, trimeric, tetrameric, etc.) form and that said activity may decrease due to loss of interaction between the subunits. For example, it has been found that the enzymatic activity of Actinoplanes missouriensis glucose isomerase is unique to the tetrameric structure. This activity is substantially lost upon dissociation of the native tetramer structure into dimers.

Therefore, the present invention provides novel mutant glucose isomerases according to claim 1 in which the interaction between subunits (monomers, dimers, etc.) is enhanced resulting in improved properties of the enzymes, especially under application conditions.

The stabilization of the tetrameric structure of glucose isomerase is achieved by strengthening the interaction between the dimers in the tetramer.

A suitable method to improve the stability of the tetrameric structure is, for example, the introduction of ionic bridges. Electrostatic effects are well known to play a fundamental role in enzyme function and structures (see J.A. Matthew et al., CRC Critical Reviews in Biochemistry, 18 (1985) 91-197). They account, for example, for the pH dependence of enzyme catalysis since the optimum pH for a given protein is determined by the presence of ionizable groups surrounding the active site. Electrostatic interactions involved in hydrogen bonds and ionic (salt) bridges are important in stabilizing the overall protein structure (see A.J. Russell and A.R. Fersht, Nature 36 (1987) 496-500). According to the assumption that the dissociation of the glucose isomerase tetramer into dimers is mainly responsible for the enzyme denaturation, this process can be prevented by introducing, in the interfaces, additional stabilizing interactions such as supplementary salt bridges.

To introduce new salt bridges in a given protein, one possibility is to substitute two neighbouring residues into oppositely charged amino acids like, for example, Asp and Arg and then check, for example by energy map calculations, that an ionic interaction could be formed. This introduction of a new salt bridge requires two point mutations. Alternatively, one may create a salt bridge by substituting a residue close to an isolated charged amino acid, thus creating an ionic pair by a single point mutation.

Another suitable method to improve the stability of the tetrameric structure is the introduction of disulfide bridges. Disulfide bonds are a common feature of many extracellular proteins. The role of these cross-linkings is mainly to stabilize proteins, this effect being one of the best understood. It is commonly explained by a decrease of the entropy of the unfolded state, but several facts remain unexplained by such a simple description. T.E. Creighton, Bioessays 8 (1988) 57-63 shows that the perturbation introduced in the native state must also be taken into account.

Many attempts have been made and reported in the literature to engineer disulfide bridges, with varying success. Pantoliano et al., Biochemistry 26 (1987) 2077-2082, having introduced two cysteine residues into subtilisin via point mutations in the gene, obtain a 3°C increase of the melting temperature. J.E. Villafranca et al., Biochemistry 26 (1987) 2182-2189, have introduced a cysteine residue by point mutation into dihydrofolate reductase and taken advantage of the presence of a free cysteine in the wildtype enzyme to form a disulfide bridge. This disulfide bond, however, has an unexpected effect, since the mutant enzyme has no increased resistance to thermal denaturation, but is more resistant to guanidine hydrochloride denaturation. In these two cases, the mutated enzymes are not as stable as expected.

These two common ways are suitable for making either a double or a single point mutation, as illustrated above. For multimeric proteins (enzymes) composed of monomeric units related by symmetry axis, a third possibility may exist: create a disulfide bridge by a single point mutation without any requirement of a free Cys. This method was successfully used by Sauer et al., Biochemistry 25 (1986) 5992-5998, in lambda-repressor: they obtained a 10°C increase of the melting temperature, a 60% increase of resistance towards urea denaturation and moreover the binding constant was improved.

As a prerequisite for the latter method a symmetry axis is needed between at least two monomers. A symmetry axis being also a rotational axis, one of the properties of these axes is that the distance between one point and the axis during rotations is maintained. Thus, a point mutation introduced near the symmetry axis of a multimeric structure will be reproduced closely to this point. This method offers the advantage of introducing an intermonomer disulfide bridge by a single point mutation.

An enhanced stability of the tetrameric structure of glucose isomerase is achieved by stabilizing the dimeric structure. This can be envisioned as the denaturation of the enzyme being an at least partly, reversible reaction of the tetramer into the dimers, and a subsequent dissociation of the dimers into two monomers each. By stabilizing the dimeric structure, the denaturation of the dimer will progress more slowly, and consequently reassociation into a tetramer will improve. Substitutions which reduce the susceptibility of the monomeric and/or dimeric structure for chemical modification may also have a stabilizing effect since they undo the irreversibility of the unfolding of the protein.

The tetrameric structure can be indirectly stabilised by stabilising the dimeric structure or even the monomeric structure of glucose isomerase by special mutations. By changing the packing of the monomeric and/or dimeric structures the conformational freedom of each of the parts of the tetrameric structure is decreased which causes a stabilizing effect on the tetramer.

It will be clear that mutations stabilizing the interactions between enzyme subunits will also be able to stabilize interactions between (folding) domains within a monomeric protein. For a more detailed explanation of the terms subunits and domains, reference is made to our copending application PCT/EP89/00839, which has also been filed on July 17, 1989.

It will also be evident to those skilled in the art that, if it is desired to provide for glucose isomerases with decreased stability, the stabilising forces as described above can be weakened by applying similar processes. Mutants with such properties and processes for obtaining such mutants form also part of this invention.

In the present specification both the three letter and the one letter code for amino acids is used. This code is explained in the following Table 1:

**Table 1**

| | | | | | |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamic acid | Glu | E | Serine | Ser | S |
| Glutamine | Gly | G | Tryptophane | Trp | W |
| Histidine | His | H | Tyrosine | Tyr | Y |
| Isoleucine | Ile | I | Valine | Val | V |

The invention provides also for a method of improving the interaction between glucose isomerase subunits, which is based on the insight of the three-dimensional ("3D") structure of the molecules. Information on the 3D structure of the enzyme (or enzyme complex) is of great importance to be able to make predictions as to the mutations which can be introduced.

Gross structural data have been reported for glucose isomerase of Streptomyces rubiginosus (Carell et al., J. Biol. Chem. 259 (1984) 3230-3236), Streptomyces olivochromogenes (Farber et al., Protein Eng. 1 (1987) 459-466, and Arthrobacter (Henrick et al., Protein Eng. 1 (1987) 467-475.

Although no amino acid sequence data are available for these enzymes the 3D-structural homology with Actinoplanes missouriensis glucose isomerase is striking (see F. Rey et al., Proteins 4 (1988) 165-172). To show the general applicability of the method disclosed in this specification the genes for glucose isomerase originating from various species have been cloned and sequenced. The amino acid sequences of glucose isomerases as deduced from the genes of Streptomyces violaceoruber, Streptomyces murinus, Arthrobacter spec. and Streptomyces thermovulgaris are shown to be homologous. Published amino acid sequences for the glucose isomerases of Ampullariella sp. (Saari, ibid.) and Streptomyces violaceoniger (Nucl. Acids Res. 16 (1988) 9337), deduced from the nucleotide sequences of the respective genes, display a close homology to Actinoplanes missouriensis glucose isomerase. In addition, WO 89/01520 discloses that the amino acid sequence of Streptomyces rubiginosus glucose isomerase is homologous to Ampullariella sp. glucose isomerase.

In a preferred embodiment of the invention the modified glucose isomerase enzyme shows at least 65% homology with the wild-type amino acid sequence of the glucose isomerase enzyme from Actinoplanes missouriensis.

Despite the absence of 3D structural data for most glucose isomerases, it can be concluded that all glucose isomerases from Actinomycetales have a similar tetrameric organisation.

Using the techniques as described above or other techniques known to people skilled in the art, several point mutations can be made in order to enhance the stability of the tetrameric glucose isomerase structure. (For structural data of the Actinoplanes missouriensis glucose isomerase, see F. Rey et al., ibid.). Among these, the following mutations are concerned with the stability of one monomer subunit:
- substitution of Gly residues into other residues (α-helix B) with the aim of decreasing the entropy of the unfolded state
- excision of a flexible loop (between α-helix G and β-strand H) for reducing the hydrophobic exposed surface
- introduction of a Pro residue at the beginning of β-strand E) (decrease of entropy of the unfolded state)
- mutation into Phe (α-helix G) in order to form an aromatic cluster to put an extra-contribution in the protein structure stabilization.

In a preferred embodiment of the invention substitution of arginine for lysine is performed at sites in the glucose isomerase molecule, stability of which is sought to be increased. Both residues have to be sterically accommodated in the 3D-structure of the protein.

In proteins, lysine residues, but not arginines, are prone to chemical modification. Thus, the epsilon amino group in lysine is known to react with aldehydes and ketones to generate Schiff base adducts and further modification products, which eventually results in the loss of biological activity (see e.g. P. Higgins & H. Bunn, J. Biol. Chem. 256 (1981) 5204-5208). In particular, in glucose isomerase application where high concentrations of glucose and fructose are present at elevated temperatures, such chemical modifications are an important factor in enzyme inactivation. Where lysine residues occur within interfaces between domains and/or subunits, chemical modification at such sites is likely to promote domain or subunit dissociation and/or to hamper the correct reassociation of the subunits and/or domains which are consequently irreversible trapped in the dissociated state. At such sites mutations of lysine residues to arginine residues will eliminate chemical modifications involving the epsilon amino group of lysine. Reference is made in this connection to our copending patent application PCT/EP89/00839, also filed on July 17, 1989.

By substituting arginine residues for specific lysine residues in glucose isomerase, the extent of chemical modification and its effect on enzyme activity and/or stability is reduced. Consequently, a change of a lysine residue to arginine will improve the stability of glucose isomerase during application.

Also, at sites which sterically accomodate the lysine to arginine mutations, a substitution of the arginine residues for the lysine residues will still result in an increased stability of the protein. Because the side chain flexibility for arginine is less than for lysine, due to the presence of the guanidinium group, the mutation of lysine to arginine is favored on entropic grounds. In addition, the guanidinium group is capable of forming more hydrogen bonds with neighbouring residues in the protein also leading to improved stability.

In still another preferred embodiment of the invention, particularly where enhancement of thermal stability of glucose isomerase is sought, at least one lysine residue occurring initially and particularly at a location of the type defined hereinafter is changed to arginine. The substitution of arginine for lysine will improve the electrostatic interactions in which the substitute arginine residue then participates, particularly interactions within the interface between subunits and/or domains. In this embodiment, the lysine residue to be replaced should preferably comply with the following requirements with respect to the folded, native, protein conformation:
1. The residue to be replaced should be directly involved in electrostatic interactions, preferably in the interface between subunits and/or domains.
2. The mutation should occur at a site that can sterically accommodate the amino acid residue that is introduced.
3. The residue should occur at a site of low solvent accessibility and, preferably be part of an interface between subunits and/or domains.

Preferably, one should search for amino acid residues in glucose isomerase which, while fulfilling criteria (1) and (2), have the lowest accessible surface area ("ASA") in the protein, simultaneously requiring that the ASA has a value lower than the average determined for the given residues. In sites which satisfy these prerequisites, arginine, as compared to lysine, provides an improved electrostatic interaction due to the physical-chemical properties of its side-chain guanidinium group (see e.g. D. Wigley et al., Biochem. Biophys. Res. Comm. 149 (1987) 927-929).

It is generally desired to retain a substantial amount of enzymatic activity of the mutant glucose isomerases, modified as taught by the present invention. To retain such enzymatic activity, the amino acid residues that are to be replaced should preferably not be those that have been identified as catalytic residues or as being substantially involved in cofactor binding.

It is clear that a specific amino acid substitution in accordance with the present invention can modify the stability of glucose isomerase by the combined effects mentioned above, e.g. effects such as changing the strength of an electrostatic interaction, changing the number of hydrogen bonds with neighbouring residues, by changing the conformational entropy of the enzyme or by influencing the extent of chemical modification.

A mutant glucose isomerase according to the present invention can be produced by the following general procedure. First, a careful analysis of the mechanism or mechanisms involved in glucose isomerase inactivation under specific denaturing conditions is carried out. Using the knowledge obtained from this analysis, specific lysine or arginine residues can then be identified as candidates for replacement. This is done by careful examination of the 3D structure of the enzyme, determined by methods such as crystallography (H. Wyckoff et al., (1985) "Diffraction Methods for Biological Macromolecules", Meth. Enzymol. Vols. 114-115, Acad. Press), NMR analysis (K. Wuthrich, (1986) in "NMR of Proteins and Nucleic Acids", J. Wiley & sons), or, alternatively, from structure predictions based on analysis of the primary structure (for a review, see W. Taylor, Protein Engineering 2 (1988) 77), or structure derivations based on available 3D structures from homologous proteins (see e.g. T. Blundell et al., Nature 326 (1987) 347).

Finally, the substitution of the amino acid residue, located at a preferred site can be achieved by conventional methods, particularly site-directed mutagenesis of the DNA sequence encoding the glucose isomerase, using for example the vectors and procedures as described by Stanssens et al. (ibid.) and bacterial strains described by Zell and Fritz (EMBO J. 6 (1987) 1809).

The mutations discussed above are only given by way of illustration of the invention without intending to limit the scope. The invention is further illustrated by the following non-limitative examples.

Unless otherwise specified in the examples, all procedures for making and manipulating recombinant DNA were carried out by the standardized procedures described by Maniatis et al. (1982).

The following plasmids, vectors and bacterial strains, used or prepared in the Examples have been deposited in the Deutsche Sammlung für Mikroorganismen, Göttingen, West-Germany, under the provisions of the Budapest Treaty, or at the Centraal Bureau voor Schimmelcultures (CBS), Baarn, The Netherlands:

| | |
|---|---|
| pMc5-8 DSM | 4566 |
| pMa5-8 | DSM 4567 |
| pECOR251 | DSM 4711 |
| E. coli K527 | CBS 471.88 |

### Example 1

### Isolation and cloning of the glucose isomerase gene from Actinoplanes missouriensis (DSM 43046) Production of D-glucose isomerase in E. coli

D-glucose isomerase (GI) is synonymously used for D-xylose isomerase (D-xylose) ketol-isomerase, EC 5.3.1.5), an enzyme that converts D-xylose into D-xylulose. The D-glucose isomerase from Actinoplanes missouriensis produced by engineered E. coli strains is designated as EcoAmi (DSM) GI. To distinguish the Actinoplanes missouriensis gene coding for GI from the analogous E. coli xy1A gene, the former will be designated as GI.

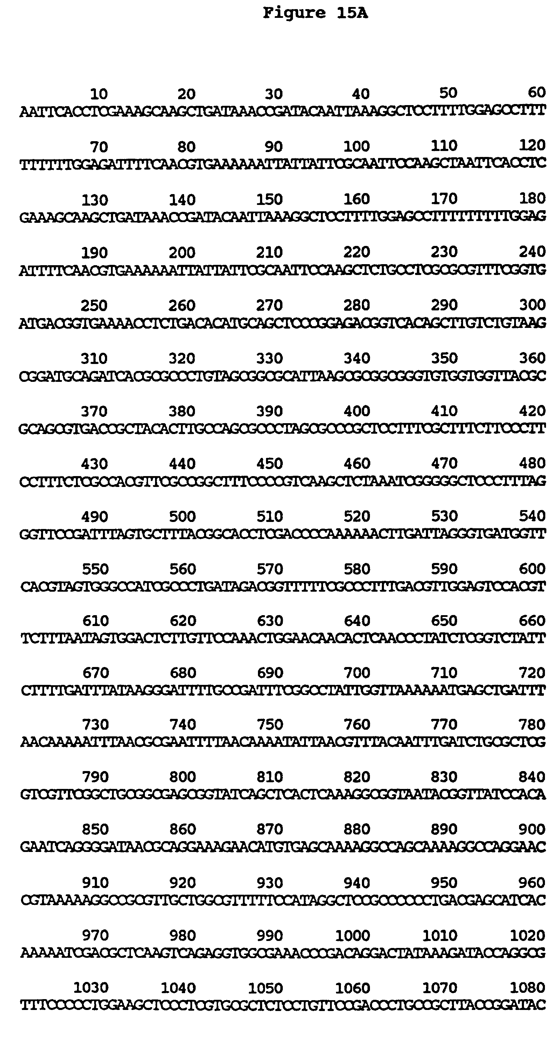

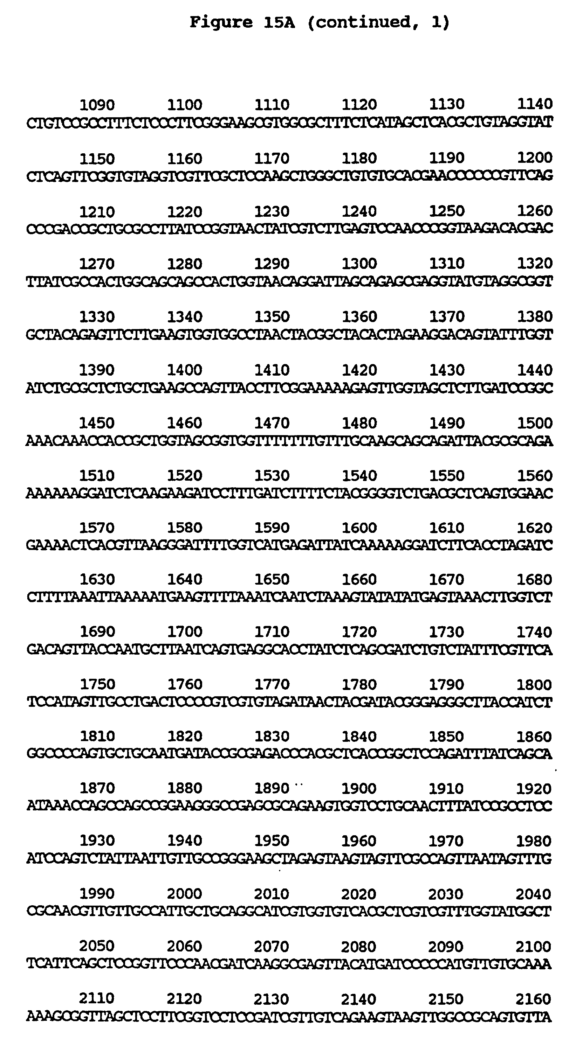

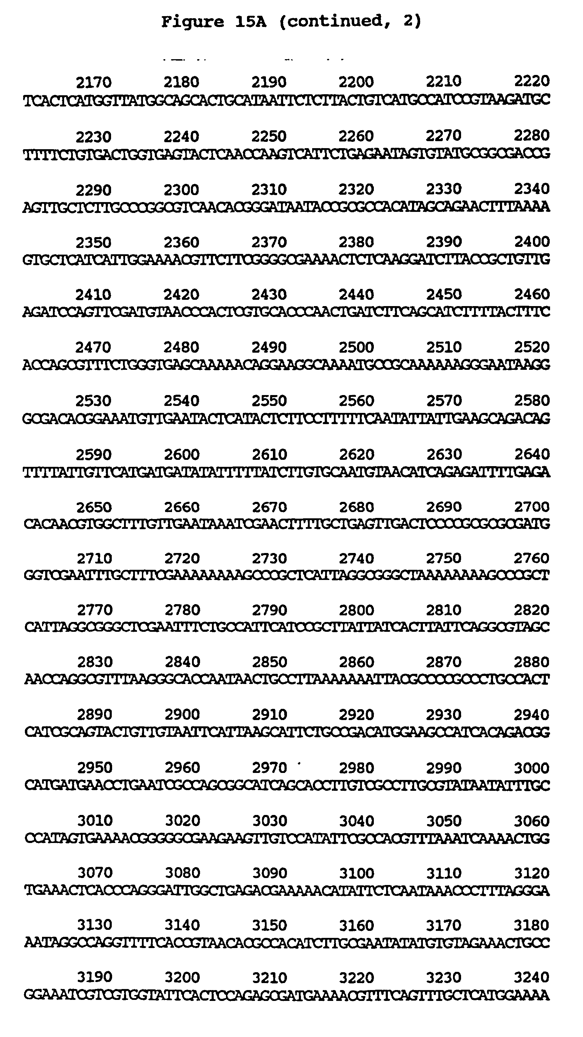

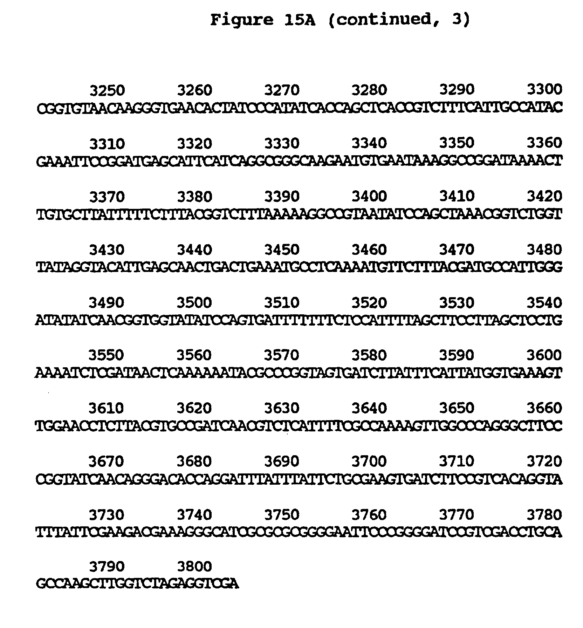

Total DNA from Actinoplanes missouriensis DSM 43046 was partially digested with Sau3A. The digest was fractionated on a sucrose gradient and fragments with lengths between 2 and 7 kilobases (kb) were ligated in the unique BglII site of plasmid pECOR251. The xylose isomerase deficient E. coli strain AB 1886 - as described in Howard-Flanders et al. (Genetics 53 (1966) 1119) and derived from E. coli strain AB 1157 (DSM 1563)- was transformed with the ligation mix and subsequently grown on minimal agar plates (Miller (1972) in "Experiments in Molecular Genetics", Cold Spring Harbor Laboratory, Cold Spring Harbor, N.Y.) supplemented with 100 mg/l ampicillin and 0.2% (w/v) xylose (MMX). Thirty seven clones were recovered (designated pAMI1-137) and grown in LB medium containing 100 mg/l ampicillin. Recombinant plasmid DNA was isolated and analyzed by restriction digests. Two groups of plasmid could be recognized, one (e.g. pAMI7) containing a 2.8 kb insert, the other (e.g. pAMI25) containing an 4.0 kb insert. An extensive restriction analysis showed that both types of inserts had a region of about 2.0 kb in common. Sequence determination of this region by the chemical degradation method (A. Maxam and W. Gilbert, Proc. Natl. Acad. Sci. USA 74 (1977) 560) revealed an open reading frame with a length of 1182 nucleotides which was identified as the coding region of GI. The nucleotide sequence of GI, together with the derived amino acid sequence are shown in Figure 15. In the following, the numbering of amino acids refer to Figure 15.

Very high expression of GI could be achieved in E. coli by placing the gene under the transcriptional control of the rightward promoter (P_{R}) of bacteriophage lambda as follows:

Plasmid pLK94 (J. Botterman and M. Zabeau, DNA 6 (1987) 583) was first modified to eliminate the PstI site in the β-lactamase gene. This was done by isolating the 880 bp EcoRI/PstI fragment of pLK70-70p (Botterman and Zabeau, ibid.) containing the N-terminal part of the β-lactamase gene, and the 1700 base pair (bp) EcoRI/PstI fragment of pLK94 containing the C-terminal part of the β-lactamase gene as well as the replication origin. Subsequent ligation of these fragments yielded pLK94p.

pAMI7 was cleaved with PstI and a mixture of two purified fragments of about 1800 bp in length, one of which contains the GI gene, were ligated into the PstI site of pLK94p. The ligation mixture was used to transform E. coli strain AB 1886. Ampicillin resistant, GI⁺ transformants were obtained by growing on MMX.

Plasmid DNA was isolated from a few selected transformants and characterized by restriction analysis. Plasmid pLK94p harboring the PstI fragment containing GI was designated as pLK94GI. The orientation of GI is such that the unique BamHI site is located about 470 bp upstream from the GTG initiation codon.

pLK70-70p was cleaved with PstI, made blunt end by DNA polymerase I (Klenow fragment) and subsequently digested with XbaI.
pLK94GI was linearized with BamHI and digested with exonuclease Bal31. Samples were taken at various times - the reaction was stopped with disodium ethylenediaminetetraacetate (EDTA) - cleaved with XbaI and analyzed by gel electrophoresis to determine the average size of the resected BamHI-XbaI fragments. Fragments, ranging in size from 1350 to 1450 bp were eluted from the gel. The fragment were ligated in pLK70-70p. E. coli K514 (C. Colson et al., Genetics 52 (1965) 1043) was transformed with the ligation mixture and ampicillin resistant transformants were selected at 37°C. The plasmid DNA, isolated from several transformants was characterized by restriction analysis. Twenty four clones containing plasmids with intact GI were retained and tested for production of EcoAmi (DSM) GI. Cultures were grown overnight at 37°C whereafter total cellular extracts were fractionated by polyacrylamide gel electrophoresis (PAGE) on 12.5% sodium-dodecyl sulfate (SDS) (U. Laemmli, Nature 227 (1970) 680). When compared to an untransformed K514 control culture, one of the clones was found to direct high level synthesis of a new protein of molecular weight 42 kilodaltons (kd) which was identified as EcoAmi (DSM) GI by Western blotting using a polyclonal serum raised against purified Actinoplanes missouriensis GI. The plasmid conferring high EcoAmi (DSM) GI production on E. coli K514 was designated as pLK70GI.

The P_{R}-GI transcriptional unit could be excised as a EcoRI-XbaI fragment, the sequence of which is given in Fig. 16. After elution from an agarose gel, this fragment was ligated in both pMa5-8 and pMc5-8 which were digested with EcoRI and XbaI, yielding pMa5-GI and pMc5-GI respectively. These vectors were found to direct equal and efficient synthesis of EcoAmi(DSM) GI while the expression level did not differ significantly from that obtained with pLK70GI.

Expression of GI could be further increased by changing the GTG initiation codon into an ATG triplet. This was done by site directed mutagenesis as described in Example 4 using the following oligonucleotide primer:

5'-GGACAGACATGGTTACC-3'

Wildtype and mutant GI enzymes were produced in E. coli strain K514 grown in a medium composed of 1% tryptone, 1% NaCl, 0.5% yeast extract, and either ampicillin (100 mg/l) for pMA type vector or chloramphenicol (25 mg/l) for pMC type vector. Cells were grown overnight at 37°C and centrifuged. The EcoAmi(DSM) GI enzyme could be purified as follows. The cell pellet was resuspended in a minimal volume of 0.05 M Tris (hydroxymethyl)aminomethane (Tris/HCl)), 0.1 mM CoCl₂, 10 mM MgCl₂, 0.2 M KCl, 5% glycerol, and 5 mM EDTA, pH 8.0, and lysozyme was added to a final concentration of 1.0 mg/ml. After standing for 20 min at 0°C, the cells were lysed using a French press, centrifuged (30 min at 23,000 g), and the supernatant diluted with an equal volume of 5% streptomycin sulfate. Incubation was maintained for 3 hours at 4°C and followed by centrifugation (30 min at 23,000 g). The resulting supernatant was heated to 70°C (except for the mutants K253Q and K100R which were heated to only 50°C) for 30 min and centrifuged again. The soluble upper phase was made 80% with ammonium sulfate. The precipitate which contained most of the enzymatic activity was collected by centrifugation, and then dissolved in 0.02 M Tris/HCl, 5 mM EDTA, 0.85 M ammonium sulfate. The subsequent chromatographic steps included Phenyl-Superose, Sephacryl S-200 HR, and finally Mono-Q HR 10/10. Importantly, the addition of 5mM EDTA to all buffers for chromatography was necessary in order to eliminate metal ions. Prior to use, the resulting enzyme was dialyzed 3 times against 200 volumes of 10 mM triethanolamine, pH 7.2, containing 10 mM EDTA (final pH is about 5.2), and again against 200 volumes of 5 mM (2-N-Morpholino)ethanesulfonic acid) (MES), ph 6.0, with 3 buffer changes. The metal content of the final enzyme preparation was determined by atomic absorption spectrometry on a Varian SpectrAA 30/40, and revealed that EcoAmi(DSM) GI was metal free; as an example, it could thus be shown that cobalt ions, which bind to the enzyme with very high affinity, accounted for only ± 1 x 10⁻⁴ mol. per mol of EcoAmi(DSM) GI monomer (when EDTA was omitted in the chromatographic buffers, the latter value increased to 0.5 mol. cobalt per mol. enzyme monomer). The purity of the EcoAmi(DSM) GI was assessed by SDS-PAGE and silver staining, and also by reversed phase high performance liquid chromatography (HPLC) on a Vydac C4 column.

The enzymatic activity of glucose isomerase was assayed as described below (1 unit of enzymatic activity produces 1.0 micromole of product -D-xylulose or D-fructose-per minute; therefore, specific activity -spa- is expressed as units per mg of GI enzymes).

The triphenyltetrazoliumchloride (TTC) assay was previously described for the visualization of D-xylose isomerases on disc electrophoresis (K. Yamanaka, Bull. Yamaguchi Med. School 18 (1971) 1). This staining method is based on the reaction of sugars with the tetrazolium salt to form formazan at room temperature; the reaction is specific for ketose at room temperature as aldose reacts only at 100°C. On gels, active xylose isomerase can thus be identified as a pink-red band. With minor modifications, this activity test was adapted for use in the Pharmacia PhastSystem. Briefly, following eletrophoresis, the native-PAGE gel was transferred to the PhastSystem staining chamber and incubated for 15 min at 50°C in 20 mM Tris/HCl, pH 7.2, with 50 mM xylose, 10 mM MgCl₂, 0.1mM CoCl₂; after washing with demineralized water 0.5 min at 4°C, the gel was immersed for 3 min at 20°C in 0.1% triphenyl-tetrazolium-chloride freshly prepared in 1N NaOH; the reaction was stopped by incubating the gel in 2N HCl for 15 min at 20°C; final wash was in water (0.5 min at 4°C).

GI activity can also be assayed directly by measuring the increase in absorbance at 278 nm of xylulose produced at 35°C by isomerisation of xylose by glucose isomerases. This assay was performed in 50 mM triethanolamine buffer, pH 7.5, containing 10mM MgSO₄, in the presence of 0.1 M xylose. Glucose isomerase final concentration in the assay was ± 0.01 mg/ml, and precisely determined, prior to dilution in the enzymatic assay mixture, by absorption spectroscopy using an extinction coefficient of 1.08 at 278 nm for a solution of enzyme of 1.0 mg/ml.

In the D-Sorbitol Dehydrogenase Coupled Assay, enzymatic determination of D-xylulose was performed at 35°C as previously described (Kersters-Hilderson et al., Enzyme Microb. Technol. 9 (1987) 145) in 50mM triethanolamine, pH 7.5, 10mM MgSO₄, and 0.1 M xylose, in the presence of ± 2 x 10⁻⁸ M D-sorbitol dehydrogenase (L-iditol : NAD oxido-reductase, EC 1.1.14), and 0.15 nM NADH, except that the incubation buffer also included 1mM ethylenebis(oxyethylene-nitrilo)tetraacetic acid (EGTA). Glucose isomerase final concentration in this assay was ± 2.5 x 10⁻³ mg/ml, and precisely determined as described above.

With glucose as a substrate GI activity can be assayed by the measurement of D-fructose produced during the isomerization reaction using the cysteine-carbazole method (CCM) which is based on the reaction of ketosugars with carbazole in acids to yield a purple product (Dische and Borenfreund, 1951).

### Example 2

### Kinetics of heat inactivation of EcoAmi (DSM) GI.

Heat-inactivation kinetics experiments were performed on the metal-free glucose isomerase with the additions described in each specific case. In brief, the purified enzyme was equilibrated in the desired buffer and the solution was drawn up into a Hamilton gas-tight syringe with a Teflon needle, that had been previously inserted into a glass mantle connected to a circulating waterbath (Lauda, RM6) set at the indicated temperature. Previous experiments have shown that temperature equilibration of the enzyme solution from 25 to 85°C is achieved in less than one minute. At appropriate times, aliquots were withdrawn into Eppendorf tubes and the heat denaturation process was quenched by cooling the samples to 0°C.

Alternatively, large samples were incubated as individual aliquots in Reacti-Vials (Pierce).

### 1. Temperature and metal dependence

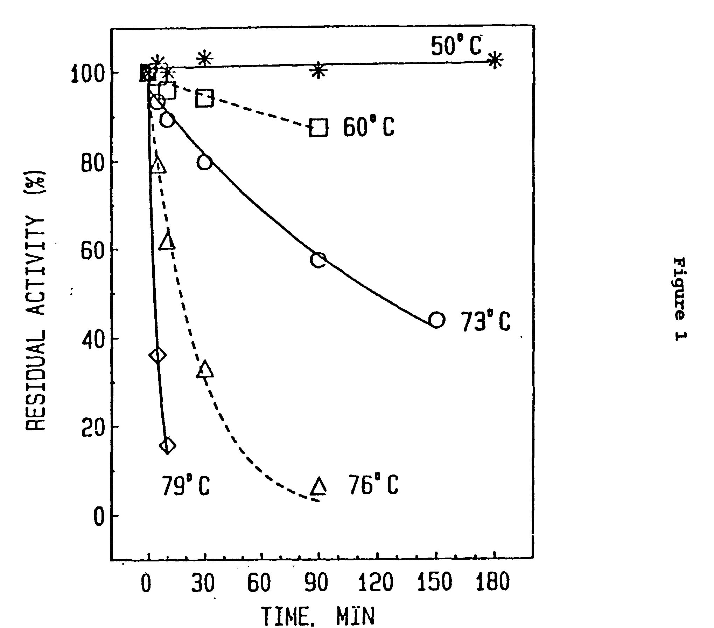

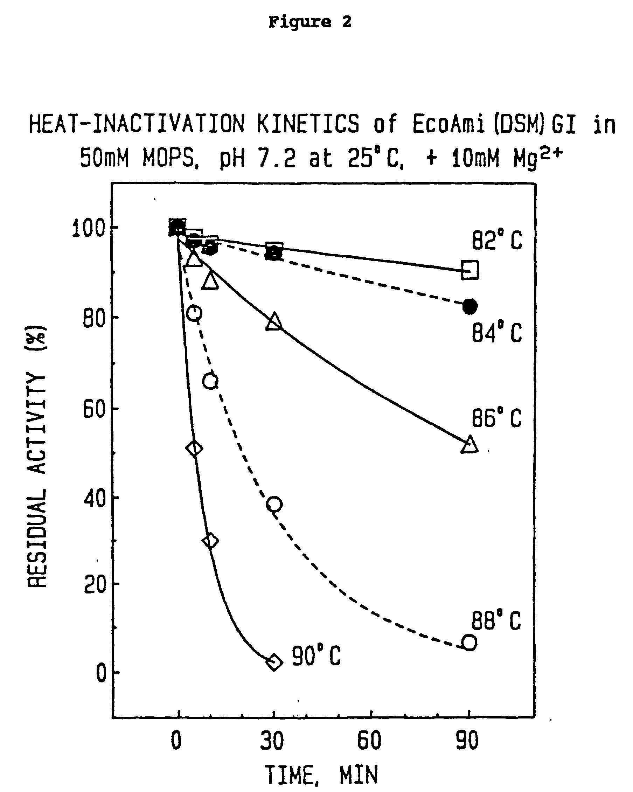

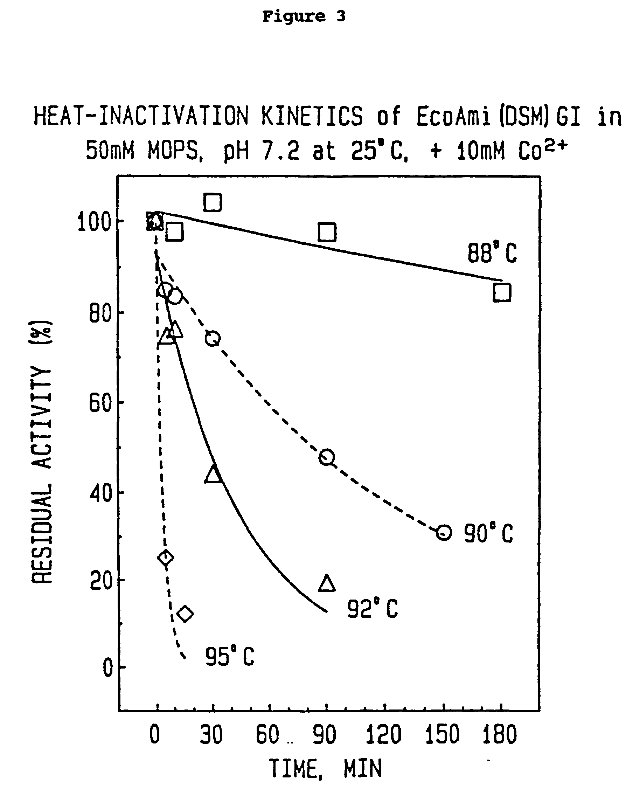

The kinetics of heat-inactivation of EcoAmi(DSM) GI in 50 mM (3-(N-Morpholino)-propanesulfonic acid) (MOPS), pH 7.2 at 25°C (pKₐ = 7.15 at 25°C; dH/°C = -0.001), as a function of temperature is illustrated in Fig. 1 (No metal added), Fig. 2 (+ 10 mM MgSO₄), and Fig. 3 (+ 10mM CoCl₂). All the data points are remarkably well fitted by theoretical decay curves corresponding to a first-order process regardless of the temperature used and of the presence or nature of the metal ion.

The data also demonstrate the stabilizing effect of magnesium and, even more so, of cobalt ions.
Enzyme inactivation by heat was found to be irreversible; accordingly, heating was shown to induce protein aggregation. In the presence of Mg²⁺, we could demonstrate that loss of enzymatic activity correlated remarkably well with the extent of protein precipitation.

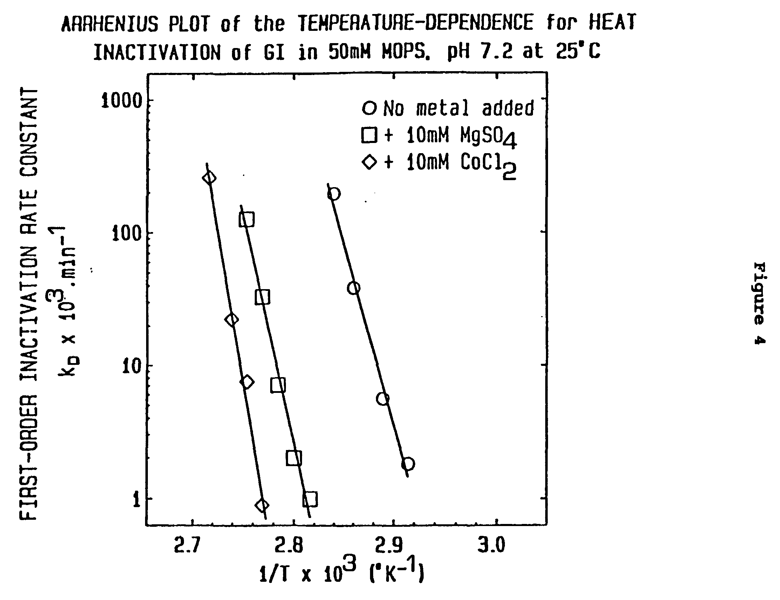

Fig. 4 summarizes the data of figures 1, 2 and 3, and shows that in the temperature intervals used the Arrhenius plot are linear whether metal is present or not.

These results indicate that thermal denaturation of EcoAmi(DSM) GI originates from one single event under all conditions tested; it is not known whether the same limiting step prevails in the absence and in the presence of metal, but the linearity of the Arrhenius plots supports the contention that this step is unique under a specific set of experimental conditions.

### 2. pH and ionic strength dependence

The affinity of GI stabilizing metals is strongly pH dependent; in particular, it is considerably reduced below pH 6. Consequently, the influence of pH on the thermostability of EcoAmi(DSM) GI was studied using the metal-free enzyme in the absence of added metals.

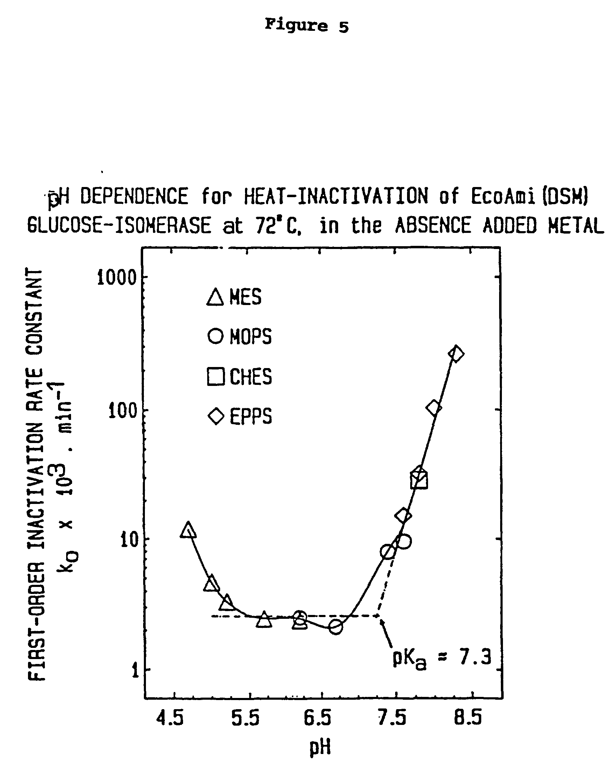

In the pH range of 4.7 to 8.3, the inactivation of EcoAmi(DSM) GI at 72°C always followed first-order kinetics. Fig. 5 shows that the inactivation rate constant, k_{D}, remained practically unaltered between pH 5.5 and 6.7, but was increased on either side of this pH range.

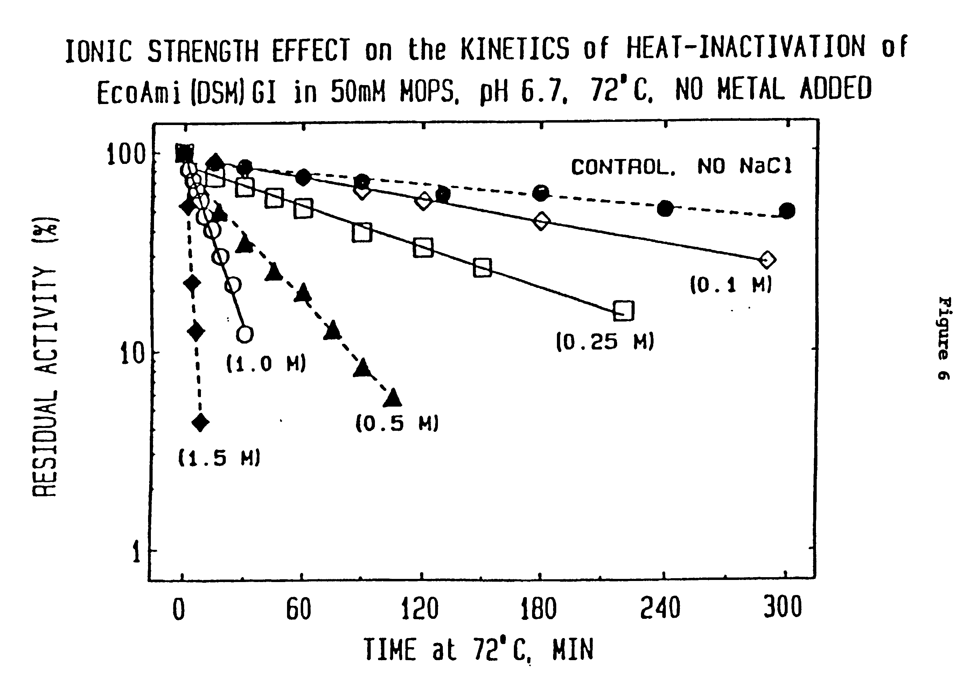

Fig. 6 demonstrates that the kinetics of heat inactivation of the enzyme in the absence of added metal was increased as a function of the ionic strength. This, together with the pH dependence data, strongly indicates that polar residues are involved in the thermal stability of EcoAmi(DSM) GI.

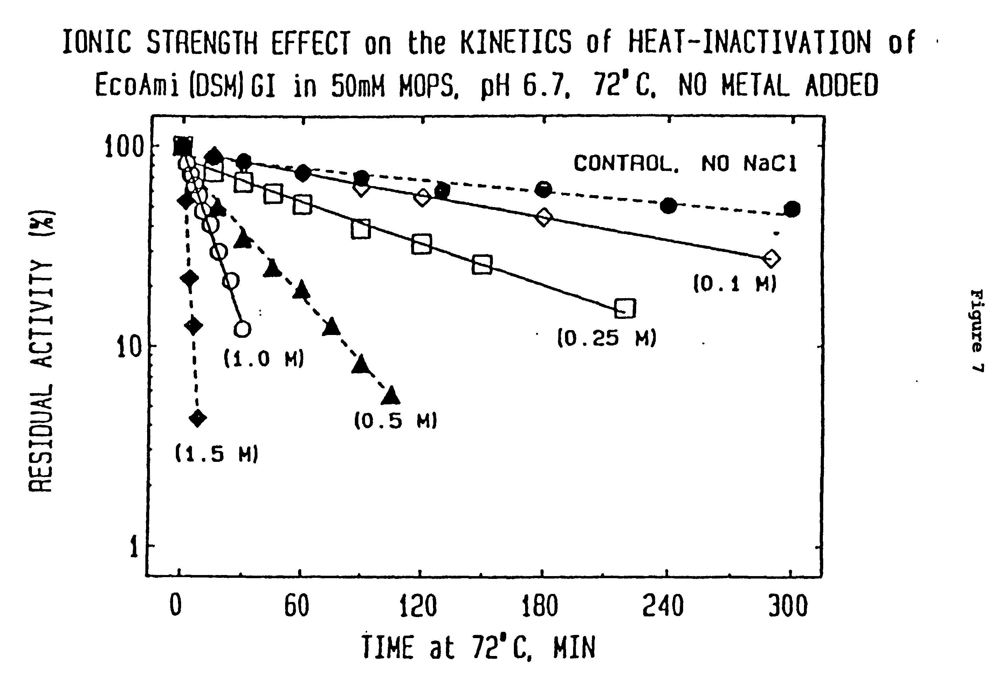

This condition is further supported by the data in Fig. 7 where it is shown that a moderate increase in pH (i.e. pH 6.7 to pH 7.6 at 72°C) significantly amplified the destabilizing effect of sodium chloride.

### 3. Effects of urea and cyanate

GI is a tetramer made of four identical subunits (F. Rey et al., ibid.).

The influence of urea and cyanate was assessed in an attempt to identify structural changes that might account for the loss of enzymatic activity as a result of heating i.e subunit dissociation and/or unfolding.

The oligomerization state of the enzyme was analysed by siz-exclusion high performance liquid chromatography (SEC-HPLC) on Superose-12 at room temperature using an elution buffer consisting of 50mM Tris/HCl pH 8.0 at 25°C and 150 mM NaCl following prolonged incubation of the enzyme in 7M urea at room temperature.
Native GI is shown to elute as a tetramer on SEC-HPLC.

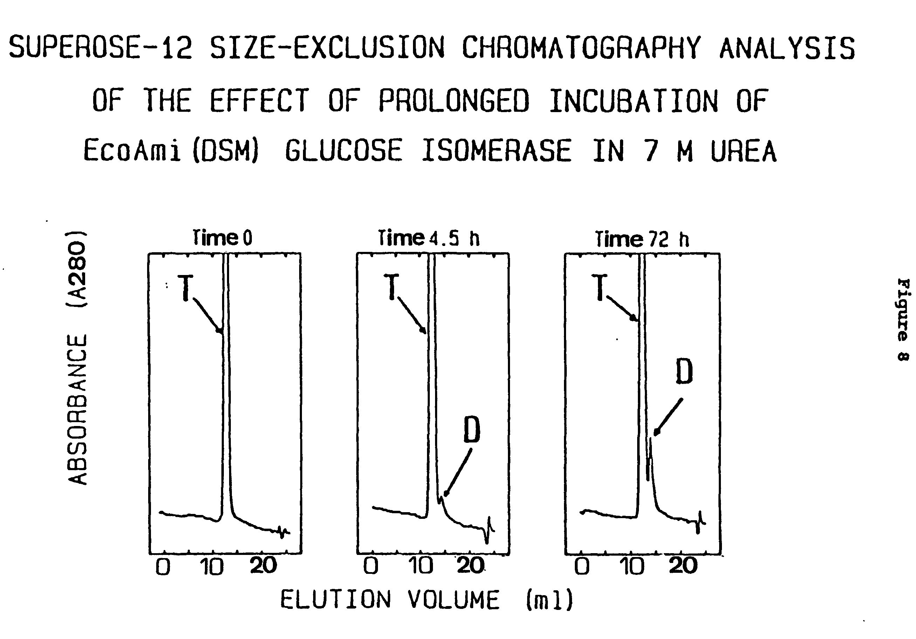

Fig. 8 shows that prolonged incubation in urea is necessary to induce a dissociation of the native EcoAmi(DSM) GI-tetramer into dimers.

Since cyanate is known to be generated from urea in solution on standing is was speculated that chemical modification of the enzyme by cyanate might be responsible for the observed subunit dissociation.
To test this hypothesis, the enzyme was incubated for 16 to 24 days at room temperature in 0.2 M borate pH 8.5 and 150 mM NaCl containing cyanate concentrations ranging from 0 to 200 mM and this in the absence or presence of freshly prepared 5.0 M cyanate freed urea (a freshly prepared stock solution of 10 M urea in Milli-Q water was passed over a column of AG 501-X8 (D) resin (Bio-Rad) according to the recommendations of the manufacturer; this treatment eliminates ionic contaminants among which cyanate).

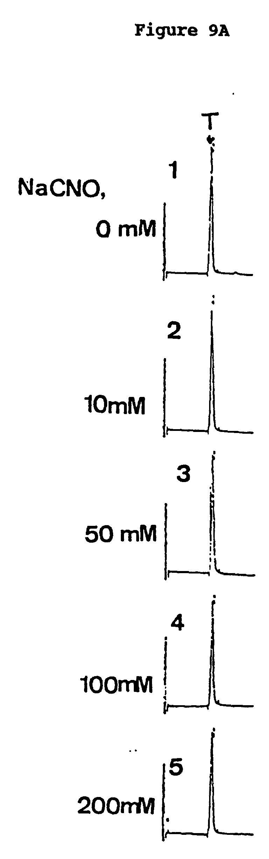

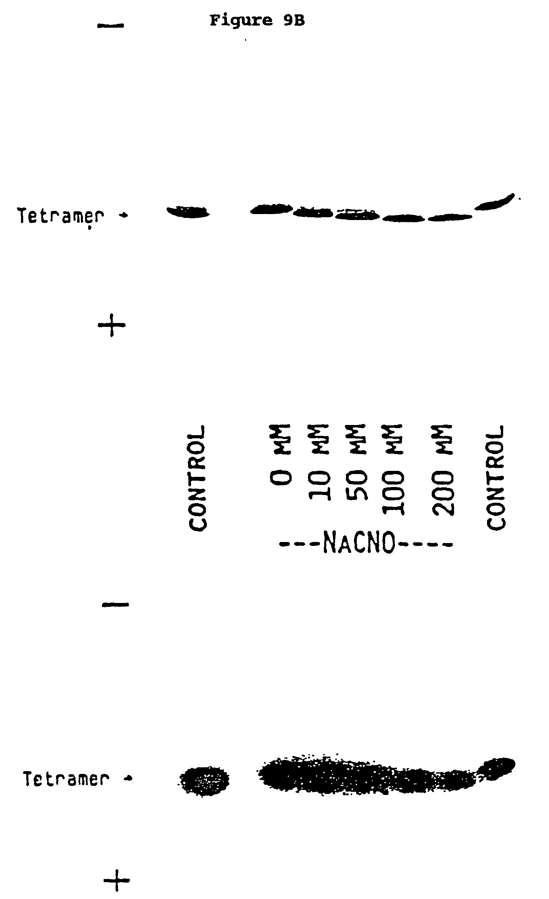

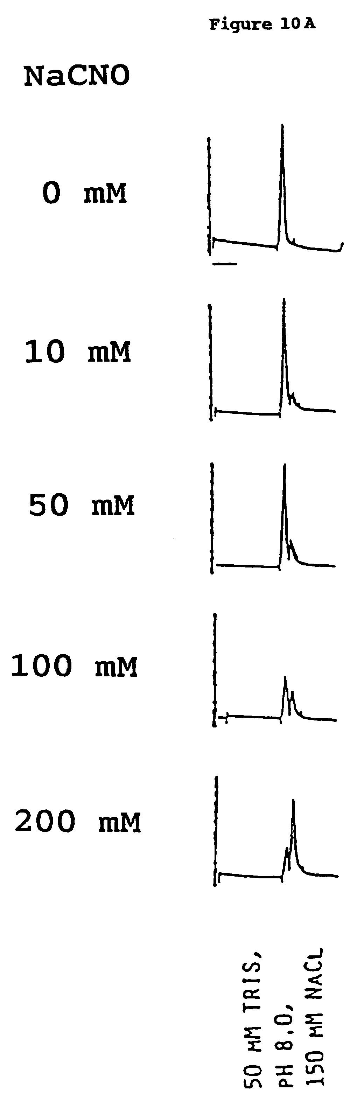

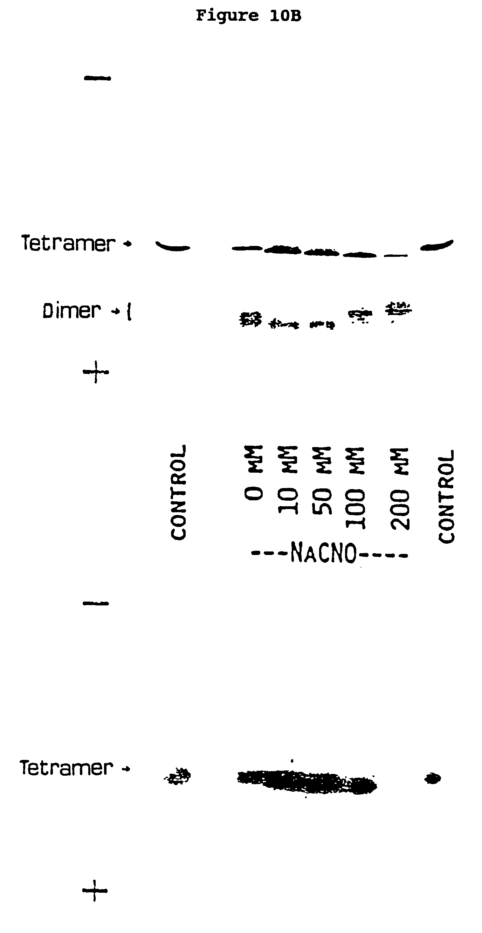

The following observations were made:
1. Treatment with cyanate alone could not alter the elution profile of EcoAmi(DSM) GI on SEC-HPLC (Fig. 9a). Native PAGE did reveal a dose dependent chemical modification of the enzyme as evidence by the increase in negative charge (Fig. 9B), upper panel) but without apparent loss of enzymatic activity as shown by TTC-staining of the gel (Fig. 9B, lower panel).
2. After 16 days of incubation of EcoAmi (DSM) GI in 5.0 M cyanate-freed urea, no dimer formation was apparent by SEC-HPLC (Fig. 10A). Some dimer-dimer dissociation however was observed after native PAGE (Fig. 10B, upper panel, 0 mM NaCNO) suggesting that at the urea concentration used (5 molar), generated cyanate induced minor chemical modification which, although not directly leading to tetramer dissociation, weakened the dimer-dimer association to an extent that dissociation could be brought forth under the influence of the electrical field applied during PAGE. Alternatively, it can also be proposed that the combined influence of urea and of the electrical field brings about tetramer to dimer dissociation.
3. The simultaneous addition of cyanate to EcoAmi(DSM) GI in 5.0 M urea readily brought about tetramer to dimer dissociation in a concentration dependent fashion. This was demonstrated both by SEC-HPLC data (Fig. 10A) and by native PAGE (Fig. 10B, upper panel). The retardation in PAGE of the dimer after incubation at high cyanate concentrations is likely to result from an increased unfolding of the enzyme under these conditions. On native PAGE, the dimers showed no GI-activity after TTC staining (Fig. 10B, lower panel). This finding suggests that enzymatic activity is lost upon GI-tetramer dissociation into dimers and/or due to chemical modification.

In conclusion, the presence of both cyanate and urea is required to observe EcoAmi(DSM) GI tetramer dissociation into dimers. Since cyanate alone was ineffective, the chemically-modified amino group(s), involved in the stabilization of the tetramer structure of the enzyme, are not solvent-accessible in the absence of urea. Urea probably destabilizes the dimer/dimer interaction, thereby exposing amino acid(s) previously buried within the dimer/dimer interface. These residues, bearing either an alpha and/or an epsilon amino group, become thus available for carbamylation by cyanate. In turn, covalent attachment of cyanate to intersubunit contact residue(s) stabilizes the dimer form of the enzyme. It can therefore be proposed that the dissociation of EcoAmi(DSM) GI tetramers into dimers is probably one of the primary events in thermodenaturation. In support of this hypothesis, it could be observed that higher protein concentrations stabilized the enzyme against denaturation by heat (data not shown).

### 4. Glycation

Protein have been shown to undergo glycation, i.e. nonenzymatic modification of alpha and epsilon amino groups by glucose and numerous other sugars. It was predicted that under application conditions (high glucose concentrations, pH 7.5, and prolonged utilization) glycation of EcoAmi(DSM) GI was likely to occur as well; in particular, if EcoAmi(DSM) GI tetramers dissociate into dimers at high temperature, one would anticipate that the same amino acid residue(s) reacting with cyanate in the presence of urea would become glycated with concomitant tetramer-dimer dissociation and possibly loss of catalytic activity.

Metal-free EcoAmi(DSM) GI was incubated in the absence of magnesium at 60°C without or with D-glucose (250mM) in 50mM MOPS, pH 7.7 at 60°C. At appropriate times, aliquots were withdrawn, cooled to 25°C and tested for residual enzymatic activity by the direct xylulose absorbance assay at 278 nm.

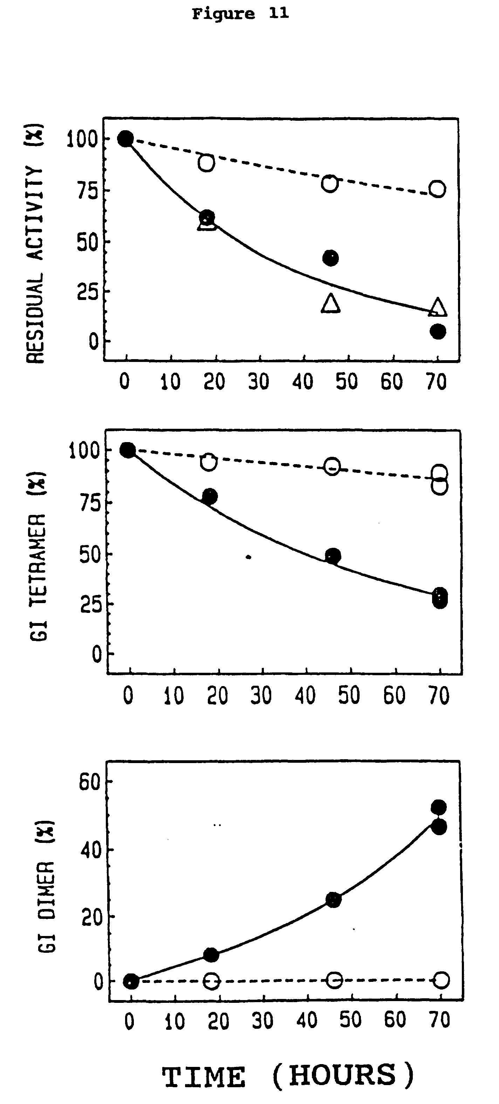

Fig. 11, Panel A, shows that the presence of glucose significantly increased the rate of heat-inactivation of the enzyme at 60°C. This effect was not reversible as extensive buffer exchange against 50mM MES, pH 6.0, at 4°C, could not restore the catalytic efficiency of EcoAmi(DSM) GI (triangles in Fig. 11, Panel A). Moreover, analysis of the reaction products by SEC-HPLC clearly demonstrated that, as predicted, glycation was accompanied by tetramer to dimer dissociation in a time-dependent fashion (Fig. 11, Panels B and C), a finding that supports the contention that tetramer splitting occurs at high temperature. Dissociated dimers are trapped by covalent modification with glucose of reactive amino groups likely to reside in the interdimer contacts.

Fig. 11, Panel D, shows that heating also caused formation of a protein aggregate having about the size of a hexadecamer of EcoAmi(DSM) GI (±700 kilodaltons, i.e. four GI tetramer molecules]; this aggregation was greatly enhanced in the presence of glucose. It is not known however whether aggregate formation occurs independently of, or only subsequent to, GI dissociation into dimers.
Very similar results could be reproduced in a different buffer system; 12.5 mM potassium phosphate, pH 7.7 at 60°C.

It is interesting to recall that the presence of urea was necessary to cyanate to produce stable GI dimers, whereas glucose exhibited the same properties at high temperature in the absence of urea. Therefore we can conclude that both urea and heating cause the dissociation of EcoAmi(DSM) GI tetramers into dimers thereby exposing amino acid residues located in the interdimer interface; among these, previously inaccessible amino groups become available to react with cyanate or glucose thus trapping the enzyme in the dimer state.

### Example 3

### Identification of lysine residues in the subunit interfaces of glucose isomerase of Actinoplanes missouriensis.

GI is a tetramer consisting of four identical subunits (A, B, C and D) (Rey et al., ibid.) which can be viewed as an assembly of two dimers (AB and CD). One can therefore distinguish two categories of subunit interfaces, interfaces between the monomers within one dimer (intra-dimer interface) and the interface between two dimers (inter-dimer interface).

A residue is said to participate in the subunit interface contacts if its accessible surface area (ASA) (B. Lee and F. Richards, J. Mol. Biol. 55 (1971) 379) calculated in the isolated subunit differs from that determined in the oligomer. Table 2 compiles the ASAs for the 20 subunit lysine residues both in the isolated monomer and the GI tetramer.

**Table 2**

| K | ASA_{T} | ASA_{T} | A_{b} |
|---|---|---|---|
| 10 | 65.4 | 65.4 | 0.0 |
| 42 | 52.6 | 52.6 | 0.0 |
| 76 | 77.1 | 77.1 | 0.0 |
| 100 | 142.4 | 142.4 | 0.0 |
| 100 | 150.0 | 0.8 | 149.2 |
| 118 | 17.9 | 6.8 | 11.1 |
| 132 | 147.4 | 147.4 | 0.0 |
| 149 | 6.9 | 3.2 | 3.7 |
| 183 | 8.0 | 0.1 | 7.9 |
| 239 | 167.0 | 167.0 | 0.0 |
| 240 | 19.2 | 18.1 | 1.1 |
| 253 | 111.5 | 1.5 | 110.0 |
| 294 | 51.5 | 28.7 | 22.8 |
| 309 | 93.2 | 93.2 | 0.0 |
| 319 | 30.0 | 30.0 | 0.0 |
| 323 | 83.0 | 83.0 | 0.0 |
| 339 | 78.0 | 50.3 | 27.7 |
| 344 | 178.1 | 125.8 | 52.3 |
| 375 | 132.0 | 119.2 | 12.8 |
| 381 | 114.2 | 67.7 | 46.5 |

Eleven of these residues are seen to participate in subunit interfaces. Only LYS-100 and LYS-253 bury an extensive area (149Å² and 110Å², respectively) in the subunit interfaces and become almost completely buried in the tetramer. In other words, both of these residues have low solvent accessibility in the tetramer. Also, neither residue is implied in the catalytic activity of EcoAmi(DSM) GI. Furthermore, LYS-100 and LYS-253 are involved in electrostatic interactions in the subunit interfaces. LYS-100 in the S-subunit (A-LYS-100) stabilizes, through hydrogen bonding the last turn of a small helix near position 373 in the B=subunit. LYS-253 in the A-subunit (A-LYS-252) on the other hand is involved in ionic interdimer interation with ASP-190 of the C-subunit. Using model building techniques (as described in P. Delhaise et al., J. Mol. Graph. 3 (1984) 116) it was observed that the environment of LYS-100 is not likely to accomodate a substitution to ARG, whereas the mutation of LYS-253 to ARG would be sterically possible because no bad physical contacts were apparent and ionic interations with ASP-190 remained favorable.

Another lysine residue, K294, is located at the dimer-dimer interface but is only partly buried in the tetramer (accessible surface area 22.8 Å²). This residue is strictly conserved in all Actinomycete glucose isomerases (see Example 10 and Fig. 21); however K294 interacts also with N247 and D257, both of which are involved in metal binding. K294 thus will affect the stability of glucose isomerase by different mechanisms.

### Example 4

### Amino acid replacements of specific lysine residues in glucose isomerase of Actinoplanes missouriensis

According to the present invention, the substitution of LYS-253 with arginine would stabilize the electrostatic interaction across the dimer-dimer interface and thereby increase the stability of EcoAmi(DSM) GI towards thermal inactivation. Moreover, this substitution would also prevent chemical modification (by glucose or cyanate) at position 253.

To assess the importance of electrostatic interactions of the A-LYS-253/C-ASP-190 ion pair in the heat stability of EcoAmi(DSM) GI, LYS-253 was mutated into glutamine to eliminate the ionic character of the lysine side-chain, this mutation being otherwise reasonably conservative.

Site directed mutagenesis was performed according to the gapped duplex DNA (gdDNA) method using the pMa5-8 and pMc5-8 like phasmid vectors (P. Stanssens et al. ibid.). Since the mutagenesis strategy requires the use of unique restriction sites upstream and downstream of the region to be mutagenized, two additional cleavage sites were introduced in the GI coding sequence without altering the encoded amino acid sequence. A KpnI site was created by nucleotide base-exchange of G at position 177 into A using the following oligonucleotide primer: 5'-CGAAGGGTACCAGG-3' A XhoI site was created by substitution of a C for a G at position 825 using the following oligonucleotide primer: 5'-GCCGTTCTCGAGGAGGTCG-3' The conversion of the GTG into an ATG codon (see Example 1) and the creation of the KpnI site were accomplished in a single mutagnesis experiment in which the relevant enzymatically phosphorylated oligonucleotides were annealed to a gdDNA derived from single stranded pMc5-GI and the large BamHI-AatII fragment of pMa5-GI.

The XhoI site was introduced in a separate experiment; the gdDNA used was constructed from single stranded pMc5-GI and the large SacI-SmaI fragment of pMa5-GI. The three mutations were assembled in a single gene by combining the appropriate fragments of the double mutant and the XhoI mutant. The resultant triple mutant was designated as pMa-I. The complementary pMc5-I was constructed by insertion of the small EcoRI-XbaI fragment of pMa5-I, containing the P_{R}-GI hybrid gene, between the EcoRI and XbaI sites of pMc5-8.

pMa5-I and pMc5-I are the basic vectors used for the production of both the wildtype and mutant GI's. In all site-directed mutagenesis experiments, described hereinafter, use was made of a gdDNA prepared from the single stranded form of pMa5-I and a suitable fragment of pMc5-I.

### 1. LYSINE-253 --> GLUTAMINE

For the construction of the gdDNA, the large SacI-XhoI fragment of pMc5-I and the following oligonucleotide primer were used:

5'-CCTGGTCGAACTGCGGGCCG-3'

The mutant enzyme was well expressed; specific activity using xylose as a substrate was 96% that of wildtype EcoAmi(DSM) GI (Table 3).

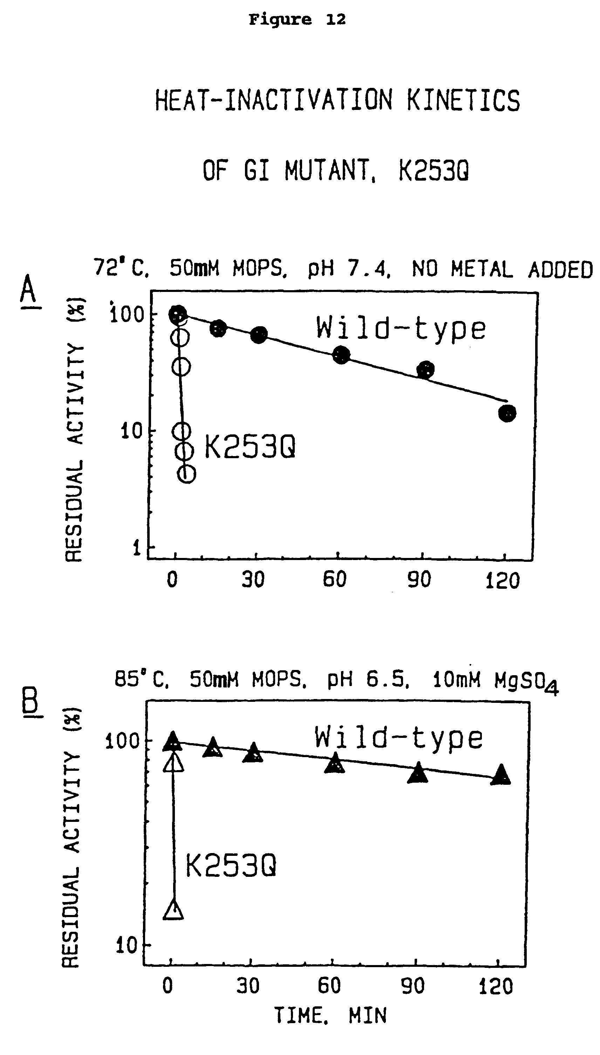

Heat-inactivation at 72°C in 50mM MOPS, pH 7.4 at 72°C, in the absence of metal, obeyed at first-order kinetics, and showed that the mutation provoked a 60-fold increase in the denaturation rate constant from 1.4 x 10⁻² min⁻¹ for wildtype to 0.9 min⁻¹ for K253Q (Fig. 12, Panel A). In the presence of 10 mM MgSO₄ at 85°C in 50mM MOPS, pH 6.5 at 85°C (Fig. 12, Panel B), the first-order decay rate constant had a value of 1.2 min⁻¹ for K253Q, about 350 times higher than that of wildtype enzyme (k_{D} = 3.4x10⁻³ min⁻¹)

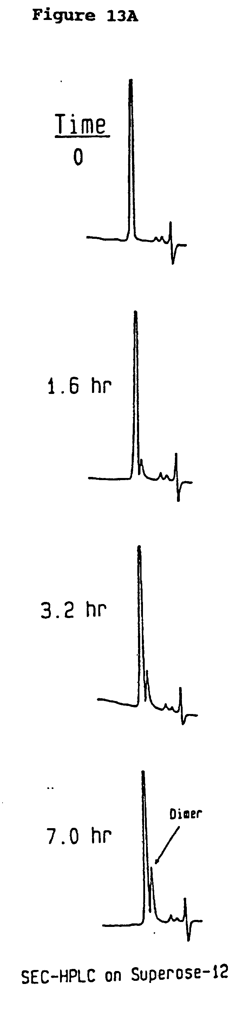

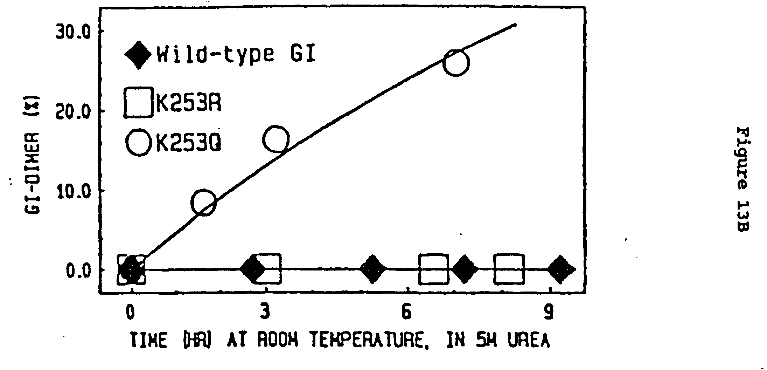

Analysis of the oligomeric structure of the K253Q enzyme by size-exclusion chromatography revealed an intact tetramer. Prolonged incubation in 5 molar, cyanate-freed, urea in 0.2M borate, pH 8.5, 0.15M NaCl, however, demonstrated that the K253Q mutant readily dissociated into dimers as shown in Fig. 13, Panel A; Fig. 13, Panel B, summarizes the data showing the progress curve for dimer formation for wildtype and mutant enzymes; the data show dissociation of tetramers into dimers in the mutant enzyme but not in the wildtype enzyme.

The experiments described above probe the structural basis of the stability of the EcoAmi(DSM) GI molecule. Specific alteration of residue K253 into glutamine introduces a temperature and also a urea sensitive mutation and consequently identifies a locus of essential interactions. A clear correlation is established between the susceptibility of the mutant to heat-inactivation and the extent of tetramer dissociation into dimers promoted by urea at room temperature.

### 2. LYSINE-253 --> ARGININE

For the construction of the gdDNA, the large SacI-XhoI fragment of pMc5-I and the following oligonucleotide primer were used:

5'-CCTGGTCGAACCGCGGGCCG-3'

The EcoAmi(DSM) GI mutant, K253R, was well expressed and displayed an enzymatic activity 120% that of wildtype's with xylose as a substrate (Table 3).

The thermostability of this mutant was tested in 50mM (N-2-Hydroxyethylpiperazine-N'-2-ethanesulfonic acid) (EPPS), pH 7.5, 5mM MgSO₄, at temperatures ranging from 82-92°C. Table 4 lists the half-lives in hours for K253R and wildtype enzymes; the results demonstrate that over this temperature range K253R is consistently more stable than wildtype enzyme.

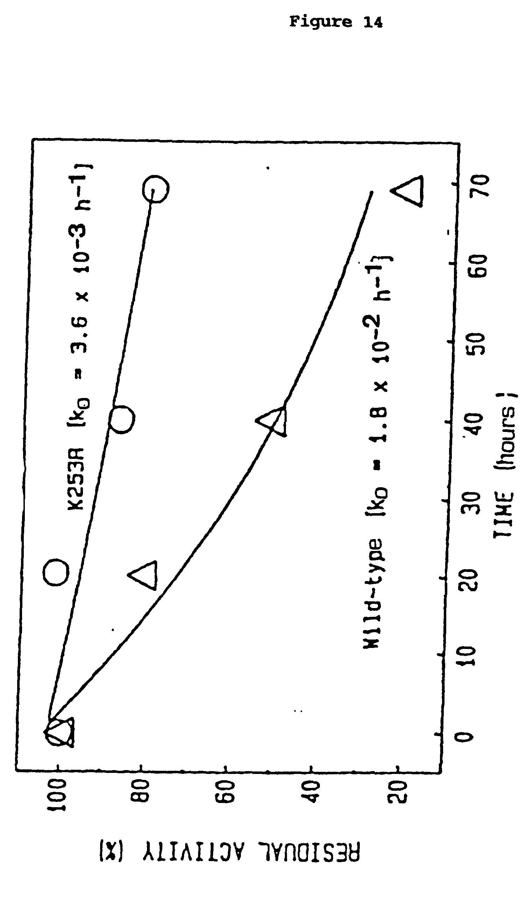

To assess the stability of the mutant K253R with regard to inactivation by glucose at high temperatures, both enzymes were incubated in the presence of 250mM D-glucose at 60°C in 12.5mM potassium phosphate buffer, pH 7.7. Shown in Fig. 14 is the time course of inactivation for about 70 hours; the data clearly demonstrate the enhanced protection against inactivating -irreversible- chemical modification achieved in the K253R mutant as its half-life is increased 5-fold compared to wildtype's.

As a negative control, LYS-100 was mutated into arginine, in which case it was expected that, as mentioned earlier, a bad steric accomodation of the new residue would lead to a decrease of stability, although without affecting enzymatic activity.

### 3. LYSINE-100 --> ARGININE

For the construction of the gdDNA, the large KpnI-AatII fragment of pMc5-I and the following oligonucleotide primer were used:

5'-CCGCCGTCCCGGAACACCGG-3'

The specific activity of the K100R GI was 22 units per mg using xylose as a substrate. It is thus comparable to the activity of the wildtype EcoAmi(DSM) GI (24.5 units per mg). Heat inactivation of this mutant enzyme, however, proceeded about 100 times faster with k_{D} = 0.3 min⁻¹, in 50mM EPPS, pH 7.5 at 84°C, 5mM MgSO₄.

### 4. LYSINE-294 --> ARGININE

For the construction of the gdDNA, the large XhoI-SmaI fragment of pMc5-I and the following oligonucleotide primer were used:

5'-GGGACGGCCGGTAGTCGAAG-3'

The EcoAmi(DSM) GI mutant, K294R, was well expressed and displayed an enzymatic activity that was 85% of the wildtype's with xylose as a substrate (Table 3).

The thermostability of this mutant was tested in 50mM (N-2-Hydroxyethylpiperazine-N'-2-ethanesulfonic acid) (EPPS), pH 7.5, 5mM MgSO₄, at temperatures ranging from 82-92°C. The half-lives in hours for K294R are listed in Table 4; the results demonstrate that over this temperature range K294R has approximately the same stability as the wildtype enzyme.

### Table 3

### Catalytic parameters of wildtype (WT) and mutant EcoAmi(DSM) GI with either D-xylose (coupled assay) or D-glucose as substrates.

Spa = specific activity in micromoles of product (D-xylulose or D-fructose) per minute (unit), per mg of enzyme.
Vₘₐₓ is expressed in units per mg of enzyme.
K_{M} is the Michaelis constant, expressed in mM.
ND = not determined.

| | Xylose | | | Glucose | |
|---|---|---|---|---|---|
| | Spa | Vmax | Km | Vmax | Km |
| WT-GI | 24.5 | 24.2 | 4.8 | 34.8 | 290 |
| K253R | 30.0 | 24.6 | 5.3 | 27.2 | 177 |
| K253Q | 23 | 15.1 | 4.4 | 29.2 | 210 |
| K100R | 22.2 | ND | ND | ND | ND |
| K294R | 20.5 | 13.8 | 4.5 | 25.8 | 187 |
| G70S;A73S;G74T | 28 | 24.9 | 5.3 | 39.2 | 235 |

**Table 4**

| Inactivation of wildtype and engineered mutants of glucose isomerase in 50 mM EPPS, pH 7.5 at 84°C, 5 mM MgSO₄. | | | | | | |
|---|---|---|---|---|---|---|
| The half-life is the time required to reduce total enzymatic activity by 50%. | | | | | | |

| Temperature (°C) | 82 | 84 | 86 | 88 | 90 | 92 |
|---|---|---|---|---|---|---|
| WT-GI | 11.85 | 3.80 | 1.07 | 0.25 | 0.080 | 0.032 |
| K253R | 17.65 | 4.17 | 1.11 | 0.32 | 0.094 | 0.037 |
| K294R | 9.39 | 1.57 | 0.43 | 0.14 | 0.056 | ND |
| G70S;A73S;G74T | 22.88 | 4.83 | 1.21 | 0.31 | 0.093 | 0.032 |

### Example 5

### Stabilisation of glucose isomerase through mutations within the monomer

Although mutations which would improve the stability of the monomer subunit, were with regard to Example 2 not very likely to affect tetramer stability of glucose isomerase some mutations in that direction have been performed. Three residues from helix B of the 8 stranded β-barrel of glucose isomerase monomer were mutated with the aim to stabilize this α-helix and indirectly stabilize the monomer. Glycine 70 was mutated into Serine, Alanine 73 was mutated into Serine and Glycine 74 was mutated into Threonine.

For the construction of the gdDNA, the large KpnI-AatII fragment of pMc5-I and the following oligonucleotide primer were used:

5'-GCCTTCTTGAAGGTCGAGATGATGGAGTCGCGG-3'

The EcoAmi(DSM) GI mutant, G70S;A73S;G74T, was well expressed and its specific activity that was 28 units per mg (115% of the wildtype) with xylose as a substrate (Table 3).

The thermostability of this mutant was tested in 50mM (N-2-Hydroxyethylpiperazine-N'-2-ethanesulfonic acid) (EPPS), pH 7.5, 5mM MgSO₄, at temperatures ranging from 82-92°C. The half-lives in hours for G70S;A73S;G74T are listed in Table 4; the results demonstrate that over this temperature range and under the conditions used, G70S;A73S;G74T is more stable than the wildtype enzyme, and also more stable than mutant K253R.

### Example 6

### Production and purification of wildtype and mutant A. missouriensis glucose isomerase for application testing

For production of A. missouriensis glucose isomerase in E. coli, use was made of the expression unit on the pMa type vector exclusively.

Transformants of glucose isomerase deficient E. coli strain K527 (thi, thr, leu, tonA, lacY, supE, xylA, r_{K}⁻m_{K}⁺), harbouring the A. missouriensis glucose isomerase gene encoding the wildtype protein or the desired mutant protein were grown in a medium composed of: yeast extract (20 g/l), Bacto tryptone (40 g/l), casamino acids (4 g/l), NaCl (10 g/l), and ampicillin (100 mg/l) for 16 hrs at 37°C. After centrifugation the cells were resuspended in a minimal volume of buffer consisting of 20mM Tris, 10mM EDTA, 50 mM glucose, pH 8.0. Lysozyme was then added to a final concentration of 1.5 g/l. After incubation at 37°C for 30 minutes, the suspension was heated to 70°C for 30 minutes. Next the suspension was cooled to room temperature and MgCl₂ and DNaseI were added to final concentrations of 20 mM and 1.5 mg/l, respectively, and incubated at 37°C for another 30 minutes. Cellular debris were precipitated by centrifugation and the supernatant dialysed against 50 mM Tris (pH 7.5) overnight.

### Example 7

### Immobilization of wildtype and mutant glucose isomerases

The enzyme solution can be immobilized on a ionic exchange resin. Before each experiment the ion exchange resins are regenerated by treatment of the resin with a 0.5 NaOH solution (> 10 bedvolumes), water (until pH < 8), a 10% NaCl solution (> 10 bedvolumes) and water (> 20 bedvolumes). Resins Lewatit MP500A (Bayer) and Amberlite IRA 904 (Röhm & Haas) were selected for the immobilisation of glucose isomerase. The adsorption of enzyme on these anion exchange resins occurs with high efficiency. There is a linear relationship between the adsorbed amount of enzyme and the activity of the application column.

10 g of the anion exchange resin (Cl⁻ form) is placed in 50 ml of buffer (50 mM Tris HCl pH 7.5). Purified glucose isomerase is added and allowed to bind overnight at 4°C in a total volume of 100 ml with 50 mM Tris.HCl buffer (pH 7.5).

### Example 8

### Application testing of wildtype and mutant glucose isomerases

The initial activity and the stability of the immobilized glucose isomerase and its mutant were determined by measuring the pump rate at 45% conversion as a function of time according to R. van Tilburg (Thesis: Engineering aspects of Biocatalysts in Industrial Starch Conversion Technology, Delftse Universitaire Pers, 1983). From this Kₒ and k_{d} can be calculated. Kₒ, a pseudo-first-order reaction rate constant is a measure for the enzyme activity. k_{d}, a first order decay constant, is a measure for the stability of the enzyme. Although the k_{d} calculation has been described for gelatin-immobilized glucose isomerase only (R. van Tilburg, ibid.), the same calculations can be used for resin-immobilized glucose isomerase. The experimental conditions were: temperature: 70°C; reactor: downflow packed bed; substrate: 3 M glucose, 3 mM MgSO₄, 3 mM Na₂SO₃; conversion: 45% fructose; pH 7.5 (measured at 35°C); Ca: ≤3 ppm. Results for k_{d} are shown in Table 5.

**Table 5**

| Decay constants for wildtype and mutant glucose isomerase, immobilized on different resins. | | |
|---|---|---|
| | k_{d} (x 10⁶sec⁻¹) Lewatit | k_{d} (x 10⁶sec⁻¹) Amberlite |
| Wildtype | 2.7 | 2.4 |
| K253R | 1.0 | 0.7 |
| K294R | 2.8 | 2.9 |
| K253Q | n.d. | 3.7 |
| (G70S;A73S;G74T) | 2.0 | 2.1 |
| N.B.: Both wildtype and mutant enzymes were produced, purified, and immobilized according to the description in Examples 6 and 7. | | |

It can be deduced from Table 5 that both mutant K253R and G70S;A73S;G74T have a significantly improved stability in the 70°C application test. These results can be translated with a fair degree of accuracy into results that will be obtained if tests are carried out at a temperature customary in industry (R. van Tilburg, ibid.).

The low performance of mutant K253Q is an indication of the importance of a basic residue at position 253, as already deduced from the in vitro experiments described in Example 4. The Glutamine mutant probably does not form any hydrogen bond at all thereby destabilising the dimer-dimer contact.

Similarly the K294R mutant has a somewhat lower stability than the wildtype enzyme in accordance with the results obtained in the in vitro experiments.

The above results show that improvement of the dimer-dimer interface contact of glucose isomerase can result in an enzyme with superior behaviour in industrial applications. It will be clear that other residues involved in interface contacts can also be selected by one of ordinary skill. Residues which are susceptible to chemical modification or which do not show an optimal hydrogen bonding may be substituted by other amino acids according to the teaching of this specification.

Surprisingly mutations which are aimed at stabilizing the monomeric subunit also show a positive effect on tetramer stabilisation, as evidenced by the k_{d} values observed for the mutant G70S;A73S;G74T.

In an attempt to rationalize these results one should bear in mind that substitution of Glycine residues in an α-helix can result in a decreased entropy of the unfolded state of a protein thereby rendering the protein more resistant to reversible unfolding and denaturation (Proteins, 1 (1986) 43-46). In doing so the susceptibility of glucose isomerase to chemical modification (and therefore irreversible denaturation) of (partially) unfolded protein might be significantly lowered, resulting in a more thermostable enzyme. Alternatively one may consider the fact that the mutations inserted in the mutant G70S;A73S;G74T are all more hydrophilic in nature and therefore likely to be favoured on the exposed surface of helix B. This can as such lead to enhanced stability of the monomer against reversible unfolding. As stated previously this can lower the susceptibility of the tetrameric protein against irreversible denaturation.

The mutations exemplified in this Example can be introduced in a similar way in all α-helices of glucose isomerase. Helix regions as determined from the 3D structure of A. missouriensis glucose isomerase (F. Rey et al., ibid.) are at positions: α1 35-47, α2 64-80, α3 108-128, α4 150-173, α5 195-206, α6 227-239, α7 264-276, α8 300-328. Substitution of Glycine residues, introduction of hydrophobic residues and introduction of Proline residues at the amino terminus of an α-helix can be envisaged.

Both engineered mutations K253R and G70S;A73S;G74T lead to improved heat stabilization relative to wildtype glucose isomerase. However, mutant K253R appears more stable than mutant G70S;A73S;G74T in the application tests contrary to the results of the in vitro experiments described in Example 4. This can be seen as a reflection of the fact that results obtained under laboratory conditions are only to a certain degree predictive for the performance of an enzyme under application conditions. Possibly the high glucose concentration used under application conditions and the glycation process, which is dependent on glucose concentration, are responsible for this phenomenon.

### Example 9

### Properties of mutant glucose isomerase at different pH

Comparative tests were carried out with mutant K253R and WT A. missouriensis glucose isomerase at different pH values. Enzyme preparations of K253R and WT were immobilized on Lewatit as described in Example 7 and subjected to an isomerisation test as described in Example 8. Conditions were as derived from R. van Tilburg (ibid.) with glucose syrups at various pH. Figure 18 shows the k_{d} values of both enzymes as a function of pH. It can be seen that mutant K253R exhibits an improved k_{d} at values below pH 7.5 up to a value of pH 5.8. Measurements were performed at least in duplicate. Thus, mutant K253R shows not only superior behaviour in industrial applications at higher temperature (higher conversion rate), but also at lower pH (better stability of the product fructose).

### Example 10

### Cloning and sequencing of glucose isomerase genes from other bacterial strains

In order to obtain amino acid sequence information on glucose isomerase from different bacteria, the genes encoding the glucose isomerase were isolated from the chromosome of the respective bacteria via molecular cloning in E. coli.

The following bacteria, several of which are known to produce industrially applied enzymes, were selected for this purpose:
- Arthrobacter species
- Streptomyces violaceoruber LMG 7183
- Streptomyces thermovulgaris DSM 40444
- Streptomyces murinus DSM 40091
- Ampullariella species ATCC 31351

Chromosomal DNA for all species mentioned was isolated and purified essentially as described by Hopwood (ibid.)

For Arthrobacter, S. violaceoruber, and Ampullariella partial digestion with restriction endonuclease Sau3AI and cloning of the resulting fragments in E. coli was performed exactly as described for A. missouriensis in Example 1. Chromosomal DNA from S. murinus and S. thermovulgaris was digested completely with PstI followed by ligation into pECOR251 as described in Example 1.

Colonies containing the desired glucose isomerase gene were detected by colony hybridization using a 712 bp MluI restriction fragment from the A. missouriensis glucose isomerase gene or a 853 bp SacII restriction fragment located within the S. violaceoruber glucose isomerase gene.

The recombinant plasmids containing the glucose isomerase genes from the different bacteria were further characterized by restriction mapping as described for the A. missouriensis glucose isomerase gene. Nucleotide sequence analysis of the protein coding regions was performed using the chemical method devised by Maxam and Gilbert (ibid.).
The clone containing the S. thermovulgaris glucose isomerase gene turned out to be incomplete: only the coding sequence upto amino acid 346 (equivalent to position 351 of A. missouriensis glucose isomerase) has therefore been established.

It should be noted that the amino acid sequence of the Ampullariella sp. glucose isomerase differs from the published sequence in that proline 177 of the published sequence was found to be an arginine.

The results of this example are summarized in figure 21, in which the amino acid sequences, derived from the established nucleotide sequences, are aligned as to maximally display the mutual homology.

### Example 11

### Expression and mutagenesis of Ampullariella sp. glucose isomerase

For expression of Ampullariella sp. glucose isomerase in E. coli use was made of the efficient expression unit already available for A. missouriensis glucose isomerase. In fact the A. missouriensis expression vector was mutated using restriction fragments from the Ampullariella sp. glucose isomerase gene covering all nucleotide and resulting amino acid differences of the coding regions of the two genes: a gapped duplex molecule was formed consisting of single-stranded DNA of pMa-I and the 4107 bp BstEII/NcoI fragment of pMc-I, a 124 bp BstEII/ApaLI fragment and a 1059 bp ApaLI/BssHII fragment, the latter two fragments being derived from the Ampullariella sp. glucose isomerase gene. The procedure was continued essentially as described in Example 1, including a renewed nucleotide sequence determination of the gene to check for unwanted alterations. Thus the correct plasmid was found and named pMc-GIampl.

The amino acid sequence comparison showed that the Ampullariella glucose isomerase, like the A. missouriensis glucose isomerase, contained a lysine residue at the equivalent position 253. Therefore a mutant of Ampullariella sp. glucose isomerase wherein lysine (K) at position 253 was replaced by arginine (R) was generated using a gapped duplex molecule formed by annealing single-stranded pMc-GIampl DNA with BamHI/HindIII digested pMa-GIamp1 and the phosphorylated mutagenic oligonucleotide: 5'-AGGTCCTGGTCGAACCGCGGGCCGTGCTGG-3'. The procedure following the annealing step, i.e. selection and analysis of the resulting mutant, was performed exactly as described in Example 1.

### Example 12

### Expression and site directed mutagenesis of Streptomyces murinus glucose isomerase

Expression of the Streptomyces murinus glucose isomerase gene was achieved by placing the gene downstream of the Tac promoter of plasmid pMaT5. pMaT5 is a derivative of plasmid PMa5P_{R}, in which the lambda P_{R} promoter is replaced by the regulatable Tac promoter (H. de Boer Proc.Natl.Acad.Sci.USA 60 (1983) 21). The structure of this expression vector is indicated in figure 15a/c.
A 1280 bp BstXI/MluI restriction fragment containing the complete Streptomyces murinus glucose isomerase gene was treated with DNA polymerase I (Klenow fragment) to convert the fragment boundaries to blunt ends, and subsequently was ligated into pUC19. Next the gene was excised again using BamHI and HindIII, and inserted into BamHI/HindIII digested pMc5T. The resulting plasmid was named pMc-GIsmul. The nucleotide and derived amino acid sequence of the Streptomyces murinus glucose isomerase and the structure of the expression unit are shown in Figures 19 and 20, respectively.

Also S. murinus glucose isomerase has a lysine residue in the protein sequence at position 253, equivalent to K253 in A. missouriensis. The mutation resulting in a substitution of lysine 253 (K) into arginine (R) in the Strectomyces murinus glucose isomerase was introduced using a gapped duplex molecule consisting of single-stranded pMc-GIsmul DNA, BamHI/HindIII digested pMa5T, and the phosphorylated mutagenic oligonucleotide 5'-GGTCCTGGTCGTACCGGATGCCGGACTGG-3' The procedure following the annealing step, i.e. selection and analysis of the resulting mutant, was performed essentially as described in Example 1.

## Claims

1. A modified glucose isomerase enzyme in which the interaction between glucose isomerase subunits is enhanced, wherein said modified glucose isomerase comprises an amino acid sequence derived from a glucose isomerase wild-type sequence in which at least one of the following amino acid substitution(s) has been performed: Lys253Arg and Gly70Ser;Ala73Ser;Gly74Thr, the amino acid positions corresponding to the numbering adopted for the amino acid sequence alignment of glucose isomerase enzymes given in Figure 21.

2. A modified glucose isomerase enzyme according to claim 1 which is derived from Actinoplanes missouriensis.

3. A modified glucose isomerase enzyme according to claim 2 in which the amino acid sequence thereof shows at least 65% homology with the wild-type amino acid sequence of the glucose isomerase enzyme from Actinoplanes missouriensis.

4. A modified glucose isomerase enzyme according to claim 2 or claim 3 derived from Actinoplanes missouriensis and having an amino acid sequence comprising the amino acid substitution Lys253Arg.

5. A modified glucose isomerase enzyme according to claim 2 or claim 3 derived from Actinoplanes missouriensis and having an amino acid sequence comprising the amino acid substitutions Gly70Ser;Ala73Ser;Gly74Thr.

6. An immobilized glucose isomerase comprising a modified glucose isomerase enzyme according to any one of claims 1 - 5.

7. Use of a modified glucose isomerase enzyme according to any one of claims 1 - 6 in the production of fructose syrups.

## Patentansprüche

1. Modifiziertes Glucoseisomerase-Enzym, in dem die Wechselwirkung zwischen Glucoseisomerase-Untereinheiten erhöht ist, wobei die modifizierte Glucoseisomerase eine Aminosäureseqüenz umfasst, die von einer Glucoseisomerase-Wildtyp-Sequenz abgeleitet ist, in welcher mindestens eine der folgenden Aminosäure-Substitution(en) durchgeführt worden ist: Lys253Arg und Gly70Ser; Ala73Ser; Gly74Thr, wobei die Aminosäure-Positionen der Nummerierung entsprechen, welche für die Aminosäuresequenz-Anordnung von Glucoseisomerase-Enzymen, die in Fig. 21 angegeben ist, angenommen worden ist.

2. Modifiziertes Glucoseisomerase-Enzym nach Anspruch 1, das von Actinoplanes missouriensis abgeleitet ist.

3. Modifiziertes Glucoseisomerase-Enzym nach Anspruch 2, in welchem dessen Aminosäuresequenz mindestens 65 % Homologie mit der Wildtyp-Aminosäuresequenz des Glucoseisomerase-Enzyms aus Actinoplanes missouriensis zeigt.

4. Modifiziertes Giucoseisomerase-Enzym nach Anspruch 2 oder 3, das von Actinoplanes missouriensis abgeleitet ist und eine Aminosäuresequenz aufweist, welche die Aminosäure-Substitution Lys253Arg umfasst.

5. Modifiziertes Giucoseisomerase-Enzym nach Anspruch 2 oder 3, das von Actinoplanes missouriensis abgeleitet ist und eine Aminosäuresequenz aufweist, welche die Aminosäure-Substitutionen Gly70Ser; Ala73Ser, Gly74Thr umfasst.

6. Immobilisierte Glucoseisomerase, umfassend ein modifiziertes Glucoseisomerase-Enzym nach irgendeinem der Ansprüche 1 - 5.

7. Verwendung eines modifizierten Glucoseisomerase-Enzyms nach irgendeinem der Ansprüche 1 - 6 bei der Herstellung von Fructose-Sirupen.

## Revendications

1. Enzyme de glucose isomérase modifiée dans laquelle l'interaction entre les protomères de la glucose isomérase est accrue, dans laquelle ladite glucose isomérase modifiée comprend une séquence de l'acide aminé dérivée d'une séquence de type sauvage de glucose isomérase dans laquelle au moins l'une des substitutions suivantes de l'acide aminé a été exécutée : Lys253Arg et Gly70Ser;Ala73Ser;Gly74Thr, les positions de l'acide aminé correspondant à la numérotation adoptée pour l'alignement de la séquence de l'acide aminé des enzymes de la glucose isomérase (voir la figure 21).

2. Enzyme de glucose isomérase modifiée selon la revendication 1 qui est dérivée d'Actinoplanes missouriensis.

3. Enzyme de glucose isomérase modifiée selon la revendication 2 dans laquelle la séquence de l'acide aminé montre une homologie d'au moins 65 % avec la séquence de l'acide aminé de type sauvage de l'enzyme de glucose isomérase d'Actinoplanes missouriensis.

4. Enzyme de glucose isomérase modifiée selon l'une quelconque des revendications 2 ou 3, dérivée d'Actinoplanes missouriensis et ayant une séquence de l'acide aminé comprenant la substitution de l'acide aminé Lys235Arg.

5. Enzyme de glucose isomérase modifiée selon l'une quelconque des revendications 2 ou 3, dérivée d'Actinoplanes missouriensis et ayant une séquence de l'acide aminé comprenant la substitution de l'acide aminé Gly70Ser;Ala73Ser;Gly74Thr.

6. Glucose isomérase immobilisée comprenant une enzyme de glucose isomérase modifiée selon l'une quelconque des revendications 1 à 5.

7. Utilisation d'une enzyme de la glucose isomérase modifiée selon l'une quelconque des revendications 1 à 6 dans la production de sirops de fructose.
